# EUROPEAN PATENT SPECIFICATION

(11) **EP 3 096 621 B1**
(45) Date of publication and mention of the grant of the patent: **11.03.2020**
(21) Application number: 15739913.0
(22) Date of filing: 23.01.2015
(51) Int. Cl.: A61K 31/7105, A61P 11/00, A61K 48/00, C12N 15/11

(54) **E. COLI MEDIATED SIRNA SILENCING OF AVIAN INFLUENZA IN CHICKENS**
E. COLI-VERMITTELTE SIRNA-SILENCING VON VOGELGRIPPE IN HÜHNERN
SILENÇAGE D'ARNSI MÉDIÉ PAR E. COLI DE LA GRIPPE AVIAIRE CHEZ LE POULET

(30) Priority: 23.01.2014 US 201461930821 P; 29.04.2014 US 201461986033 P
(43) Date of publication of application: 30.11.2016
(73) Proprietor: COLORADO STATE UNIVERSITY RESEARCH FOUNDATION, Fort Collins, CO 80522 (US)
(72) Inventor: LINKE, Lyndsey M., Windsor, Colorado 80550 (US); SALMAN, Mo D., Fort Collins, Colorado 80528 (US); WILUSZ, Jeffrey, Fort Collins, Colorado 80525 (US)
(74) Representative: Cabinet Chaillot
(86) International application number: PCT/US2015/012704
(87) International publication number: WO 2015/112873

(56) References cited:
- US-A1- 2007 099 858
- US-A1- 2010 189 691
- US-A1- 2010 204 297
- US-A1- 2013 225 651
- XIANG S ET AL: "Short hairpin RNA-expressing bacteria elicit RNA interference in mammals", NATURE BIOTECHNOLOGY, GALE GROUP INC, US, vol. 24, no. 6, 1 June 2006 (2006-06-01), pages 697-702, XP002479733, ISSN: 1087-0156, DOI: 10.1038/NBT1211
- LINKE LYNDSEY M ET AL: "Inhibiting avian influenza virus shedding using a novel RNAi antiviral vector technology: proof of concept in an avian cell model.", AMB EXPRESS MAR 2016, vol. 6, no. 1, March 2016 (2016-03), page 16, XP002773935,
- LAGE ET AL.: 'Bacterial delivery of RNAi effectors: transkingdom RNAi.' J VIS EXP. 2010, XP055215018

## Description

### BACKGROUND OF THE INVENTION

### 1. FIELD OF INVENTION

This invention relates to compositions and methods for treating or preventing avian influenza in poultry. More specifically, this invention relates to siRNA compositions that interfere with avian influenza viral replication in chickens and other poultry.

### 2. BRIEF DESCIPTION OF THE RELATED ART

Avian Influenza Virus (AIV) is a viral disease that infects specific tissues in many avian species, including tissues of the respiratory, digestive and/or nervous system. While rarely fatal in wild birds, AIV is highly contagious and often fatal when transmitted to domestic poultry (Swayne and Halvorson 2008, Capua and Marangon 2006, Horimoto and Kawaoka 2001). Highly Pathogenic Avian Influenza (HPAI) viruses rapidly infect poultry and are often fatal, with a case-fatality rate approaching 100% (Horimoto and Kawaoka 2001). Low Pathogenic Avian Influenza (LPAI) viruses typically cause mild disease that can go undetected; however, the economic effect of LPAI lies in loss of production. The risk for LPAI and HPAI outbreaks are significant threats to the poultry industry.

Current prophylactic methods for AIV in poultry are limited. Available vaccines do not confer complete immunity, as evidenced by the ability of the virus to replicate in vaccinated birds (Capua et al 2004). Vaccines rely on a healthy immune system and could be rendered ineffective due to viral antigenic evolution (Arzt et al 2010, Escorcia et al 2008, Zhou et al 2008, Bennink et al 2004, Ge et al 2004). Results from previous studies showed that AIV field isolates from poultry exhibited constant drifts in their genetic information and the persistence of the virus in the field was likely aided by antigenic differences attributed to the vaccine strain (Escorcia et al 2008, Lee et al 2004). Interestingly, some of the vaccines created for controlling HPAI outbreaks were precursors to new HPAI outbreaks. Vaccine protection can take more than one week to acquire (Kim et al 2009); therefore, vaccination during an outbreak offers little protection. Vaccination is most effective via intramuscular injection, which is not practical for large-scale vaccine administration. Finally, improper storage and handling often leads to vaccine failure. There are also growing concerns that current avian H5N1 viruses are becoming resistant to amantadine, rimantadine and oseltamivir (Cheung et al 2006, Le et al 2005, de Jong et al 2005). Reports have linked treatment with these common drugs to the shedding of drug resistant viruses. As an example, in a desperate attempt to protect their poultry farms, China was reported to have administered amantadine during the 2005 H5N1 outbreaks. This misuse has led to drug resistant strains that are circulating in China and southeast Asia (Cheung et al 2006). The lack of robust prophylactics and the endemic nature of this disease underlines the urgency to develop more effective control measures in poultry, as a means of controlling AIV transmission and reducing the impact outbreaks have on poultry operations. It is specifically valuable to consider new prophylactic strategies that can protect poultry against any subtype or strain of AIV.

US2013/225651 and US2007/099858 disclose compositions useful for modulating influenza virus gene expression using siRNAs. The document is silent about the incorporation into an E.coli bacteria as well as the presence of Inv and hlvA genes.

Xian S. et al. (Nature Biotechnology, vol. 24, no. 6, 1 June 2006, pages 697-702) disclose non-pathogenic E.coli bacteria engineered to transcribe various shRNA from plasmid containing the inv and the hlvA genee, which encode tow bacterial factors needed for successful transfer of shRNA into cells. The document is silent about interfering with the expression of Avian Influenza viral RNA molecules.

Lage et al. (J.Vis. Exp., 2010) diclose a non-pathogenic E.coli bacterium comprising a prokaryotic vector, comprising a DNA molecule encoding one or more siNRAs and a promoter, wherein the siRNAs interfere with ABCB+ gene expression, and where the E.coli expresses at least one inv gene and at least one hlvA gene. The document is silent about interfering with the expression of Avian Influenza viral RNA molecules.

US2010/204297 discloses siRNAs that target viral RNA polymerase, which inhibits Influenza A virus production. The document is silent about the use of a non-pathogenic E.coli and the present construction.

US2010/189691 dicloses a non-pathogenic E.coli vector comprising a DNA molecule encoding one or more siRNAs and a promoter, wherein the siRNA interferes with the expression of beta catenin mRNA. The document is silent about interfering with the expression of Avian Influenza viral RNA molecules.

In the absence of effective control, AIV outbreaks in poultry can be devastating and estimates of potential economic losses are enormous. This is specifically true when AIV epidemics hit areas that have a higher density of poultry farms. These areas become high-risk locations for outbreaks and often face considerable challenges to control AIV transmission, despite strict biosecurity measures and depopulation efforts. Once transmitted via respiratory secretions and feces, the incubation period for AIV may last as long as 10 days and the majority of infected poultry shed virus for 7 to 10 days, allowing the virus to circulate in a flock for a long period of time (Easterday et al 1997). This potentially long shedding period increases the transmission risk to poultry, especially within larger populations (Easterday et al 1997). Developing a powerful anti-influenza technology is a critical step to effectively manage and control the spread of this disease in poultry to minimize financial losses and risks for transmission to other susceptible species, including humans.

Current vaccination strategies for birds are limited as they do not confer complete immunity, are reliant on a healthy immune system, are susceptible to viral antigenic evolution, require individual handling of every bird in a large scale commercial poultry operation, have a limited shelf-life, and antibody protection following vaccination takes several weeks to acquire. Because of these challenges, AIV vaccination within the United States is rarely favored for prophylactic use in poultry. If an emergency vaccination program were adopted, it would offer little protection if administered during an outbreak. Furthermore, growing evidence for the emergence of drug resistance AIV variants poses a risk for the use of drug therapies, such as amantadine, rimantadine and oseltamivir. These limitations indicate a real need to develop a prophylactic that can not only protect against different subtypes and drug resistant strains of virus, but would provide transient protection should an outbreak occur and vaccination is not feasible or effective in a short period of time.

### BRIEF SUMMARY OF THE INVENTION

The invention is defined by the appended claims. The present invention provides products and associated methods for the delivery of one or more small interfering RNAs (siRNAs) to an avian cell using a nonpathogenic bacterium for the prevention or treatment of Avian Influenza Virus (AIV). The siRNA is complementary to an mRNA of the AIV NP or PA sequence. In challenge studies with chickens, the siRNA is shown to prevent the onset of clinical symptoms or reduce the severity of disease, including reducing viral titers or virus shedding.

In a first aspect the present invention provides a nonpathogenic *E. coli* bacterium that includes a prokaryotic vector. The vector is a DNA molecule encoding one or more siRNAs and a promoter to control transcription of the siRNA. The siRNAs encoded by the vector interfere with one or more Avian Influenza viral RNA molecules, thereby inhibiting replication of the AIV. In addition, the bacterium is engineered to express at least one *inv* gene and at least one *hlyA* gene. The *inv* gene product helps the bacterium to gain entry into the target cell.

In an advantageous embodiment of the first aspect the siRNA interferes with an NP, PA, PB1 or PB2 mRNA. In further advantageous embodiments the siRNA is complementary to an Avian Influenza viral mRNA sequence shared by a plurality of subtypes. Ideally, the siRNA would target a sequence conserved across most subtypes. In this manner, the siRNA would be effective against as many subtypes as possible. However, shared sequences may be more limited and they may change over time. Accordingly, siRNA can be designed to be effective against a plurality of subtypes, though not all, and a "cocktail" approach with a variety of different siRNAs can be used to cover a broad spectrum of AIVs.

In still further embodiments of the first aspect, the siRNA can be generated from a DNA sequence that includes a sequence selected from the group consisting of SEQ ID No. 1, SEQ ID No. 2, SEQ ID NO. 3, SEQ ID NO. 4, SEQ ID NO. 11, SEQ ID NO. 12, SEQ ID NO. 13 and SEQ ID NO. 14. Similarly, the siRNA can be generated from a plasmid that encodes a transcript having where the plasmid has a sequence selected from the group consisting of SEQ ID No. 5, SEQ ID No. 7, SEQ ID No. 15, and SEQ ID No. 17.

The promoter that is used to control transcription of the siRNA can be a T7 promoter, a T3 promoter or an SP6 promoter. The siRNA can targets a conserved sequence, where that targeted conserved sequence is shared by a plurality of Avian Influenza subtypes. And the prokaryotic vector is a plasmid encoding the *inv* gene and the *hlyA* gene, thereby facilitating the entry of the bacterium into the cell and the delivery of the siRNA from the bacterium from the endosome upon entry.

In a second aspect the present invention provides method for treating or reducing the risk of spreading avian influenza in a poultry. The method includes the step of administering to the poultry a composition comprising the nonpathogenic *E*. *coli* bacterium according to the first aspect. In an advantageous embodiment the composition is administered intranasally or sprayed as an aerosol suitable for inhalation. In further advantageous embodiments a plurality of *E*. *coli* bacterium populations can be administered. Where a plurality of populations are administered, each population of *E*. *coli* can be engineered to express a different siRNA molecule that interferes with one or more Avian Influenza viral RNA molecules. Through such a strategy, multiple AIV mRNAs can be targeted in a single prophylactic administration.

In a third aspect the present invention provides a pharmaceutical composition having two different nonpathogenic *E*. *coli* bacteriums to deliver two different siRNAs. In other words, the first nonpathogenic *E*. *coli* bacterium has a prokaryotic vector encoding one siRNA, wherein the first encoded siRNA interferes with one or more Avian Influenza viral RNA molecules and the second nonpathogenic *E*. *coli* bacterium has a prokaryotic vector encoding a different siRNA, where second encoded siRNA interferes with a different Avian Influenza viral RNA molecule or a different portion of the same Avian Influenza viral RNA molecule. The composition is provided in a pharmaceutically acceptable carrier. In an advantageous embodiment the first encoded siRNA sequence can include SEQ ID No. 3 and the second encoded siRNA sequence can include SEQ ID No. 4. The first and second bacteria can be engineered to express at least one *inv* gene and at least one *hlyA* gene.

In a fourth aspect the present invention provides a prokaryotic vector having one or more DNA molecules encoding one or more siRNAs, a promoter to control transcription of the siRNAs, at least one *inv* gene and at least one *hlyA* gene. The encoded siRNAs interfere with the mRNA of an Avian Influenza Virus. In an advantageous embodiment the vector according to the fourth aspect can produce an siRNA that interferes with an NP, PA, PB1 or PB2 mRNA. Additionally, the siRNA can include a sequence selected from the group consisting of SEQ ID No. 1, SEQ ID No. 2, SEQ ID NO. 3, SEQ ID NO. 4, SEQ ID NO. 11, SEQ ID NO. 12, SEQ ID NO. 13 and SEQ ID NO. 14.

### BRIEF DESCRIPTION OF THE DRAWINGS

For a fuller understanding of the invention, reference should be made to the following detailed description, taken in connection with the accompanying drawings, in which:

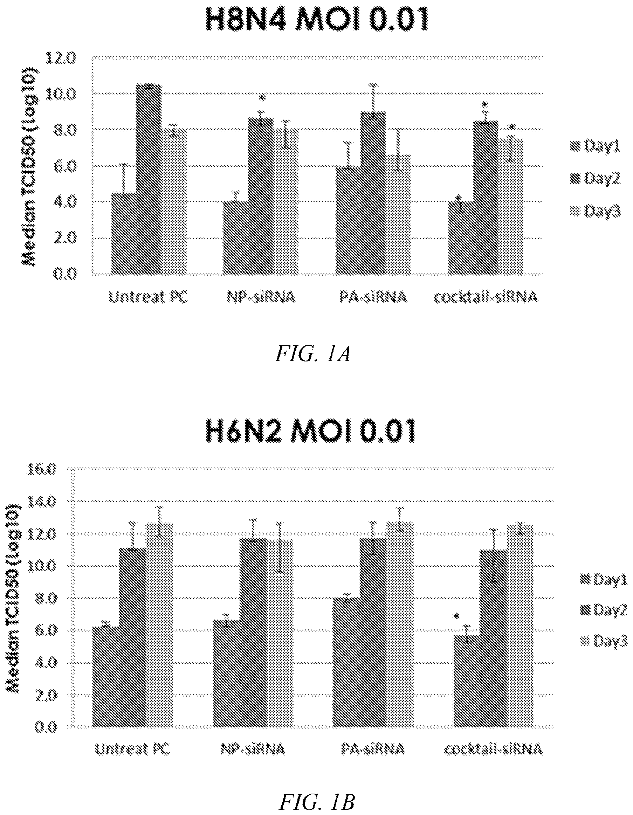

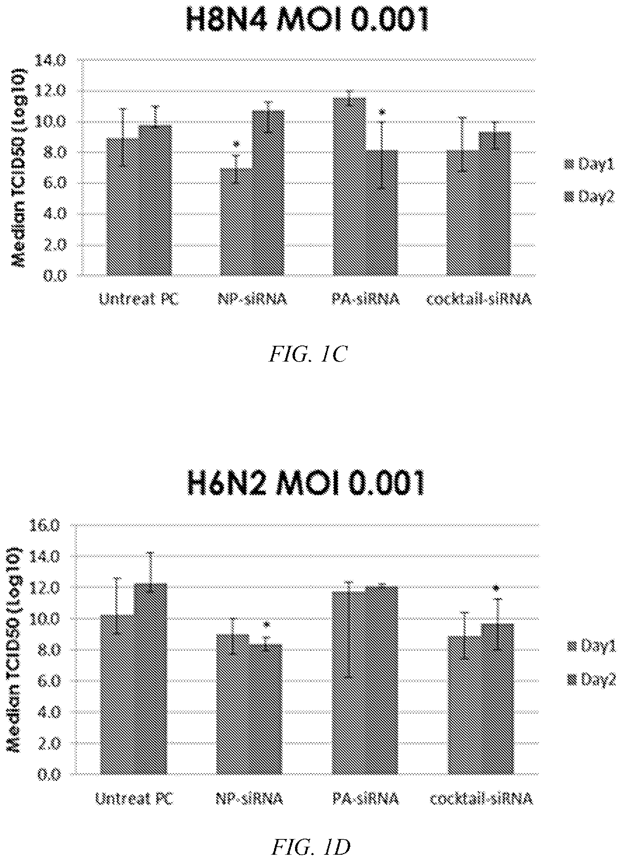

FIG. 1 is a series of four graphs (FIGS. 1A-1D) showing siRNA protection as measured by reduction in median titers by day, MOI, and virus. * indicates a statistically significant difference with corresponding untreated sample using Wilcoxon rank-sum test (p < 0.05).

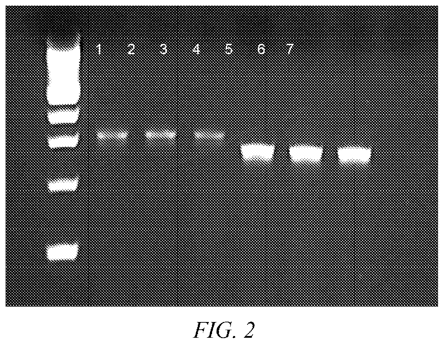

FIG. 2 is an image showing mRNA expression of β-actin (ACTB-282bp) and β1-integrin (ITGB1-308bp) in LMH cells cultured in growth normal medium (WM), infection medium (IM), or infected with A/turkey/Colorado/235497/2003(H8N4) (infected). Lanes: 1) ITGB1 in WM; 2) ITGB1 in IM; 3) ITGB1 infected; 4) ACTB in WM; 5) ACTB in IM; 6) ACTB infected; and 7) negative PCR control.

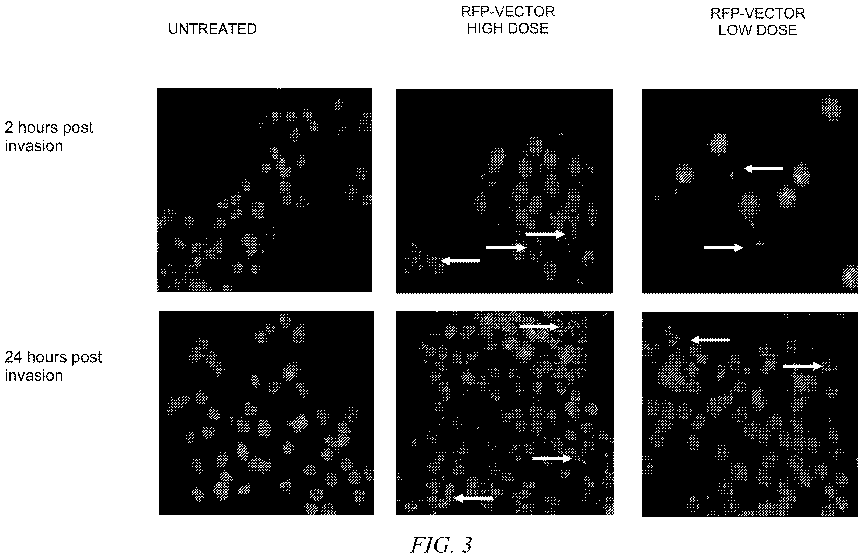

FIG. 3 is a set of six images showing anti-AIV_scramble vector tagged with RFP. Chicken LMH cells given RFP-vector at two doses (high=7.8e5 CFU/mL and low=1.95e5 CFU/mL). Vector uptake assessed at 2 and 24 hours post invasion. *Note that the white arrows point to areas of red fluorescence, which are obscured in gray scale.

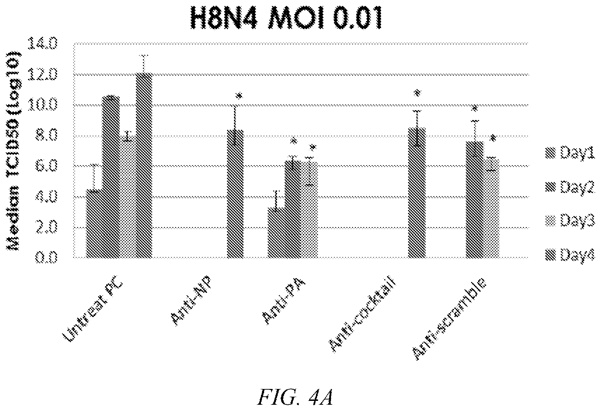

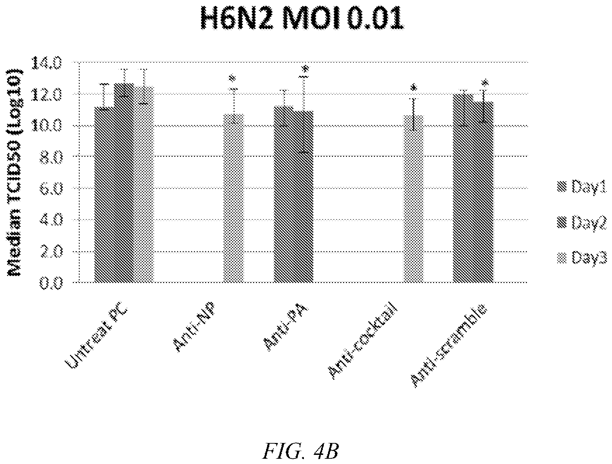

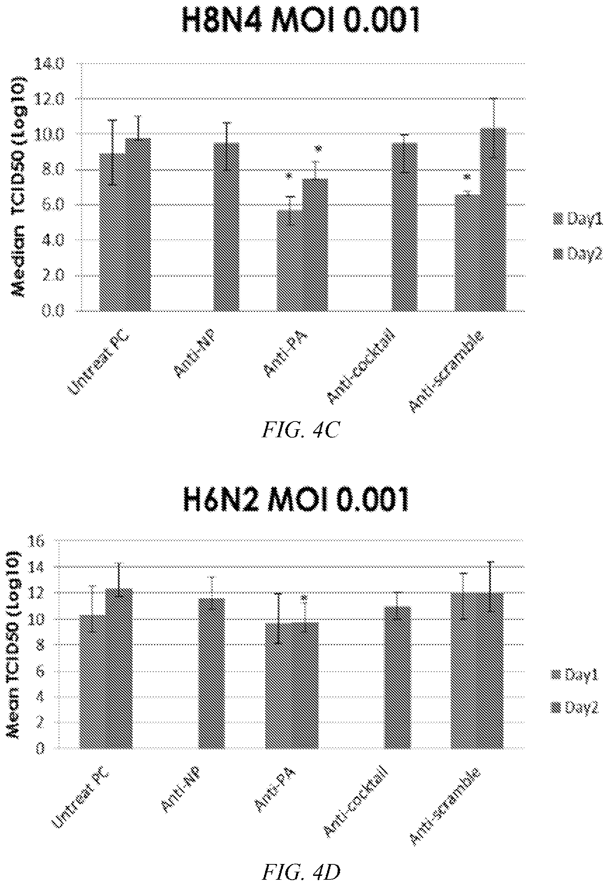

FIG. 4 is a series of four graphs (FIGS. 4A-4D) showing anti-AIV vector protection as measured by reduction in median titers by day, MOI, and virus. An asterisk (*) indicates a statistically significant difference with corresponding untreated sample using Wilcoxon rank-sum test (p < 0.05).

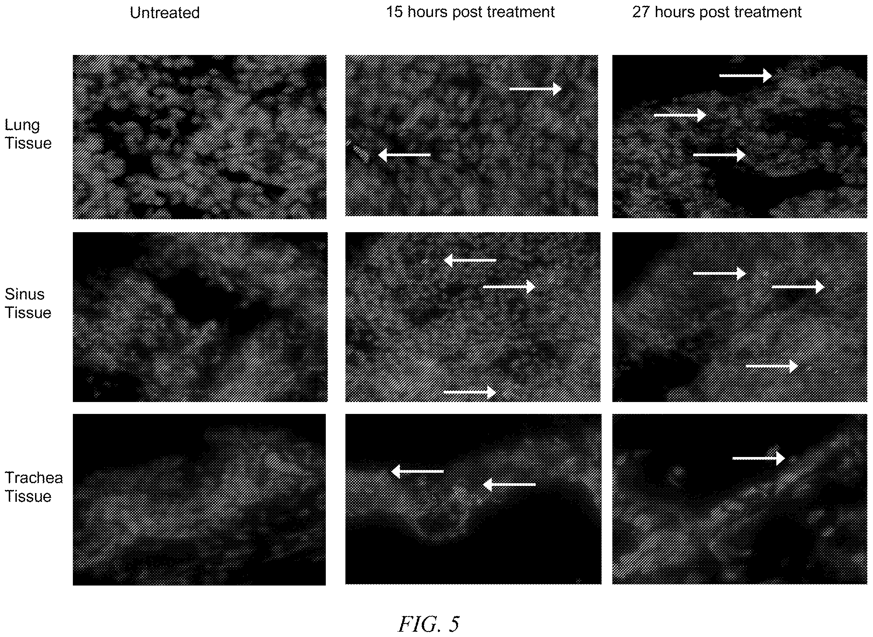

FIG. 5 is a series of six images showing anti-AIV_scramble vector tagged with RFP. Chickens were treated intranasally with RFP vector and uptake assessed at 15 and 27 hours post treatment. *Note that the white arrows point to areas of red fluorescence, which are obscured in gray scale.

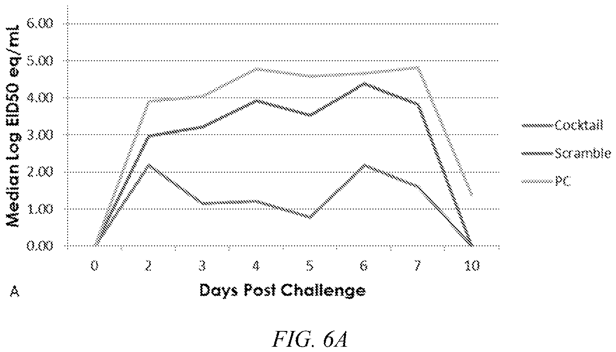

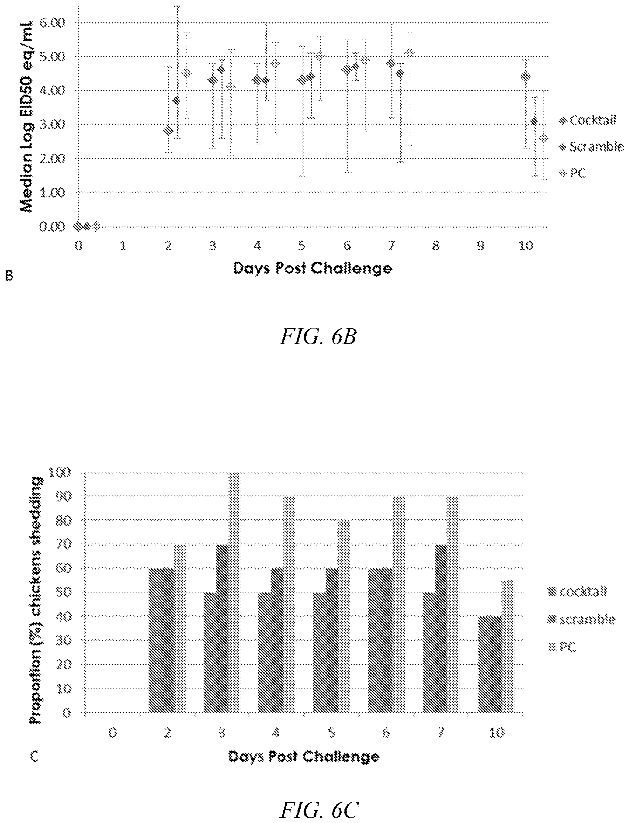

FIG. 6 is a series of three graphs (FIGS. 6A-6C) showing daily A/chicken/Texas/473-2/10(H6N2) virus titer and proportion of chickens shedding virus over the study period. (A) Median virus titer (Log EID50 eq/mL) representing all chickens (shedding and not shedding) plotted by day. The top line at day 4 is PC; the middle line at day 4 is Scramble; and the lower line at day 4 is Cocktail. (B) Median virus titer representing chickens shedding and plotted by day. For each day from left to right: Cocktail; Scramble; PC. (C) Proportion (%) of chickens shedding H6N2 virus by day, out of n=10 total chickens in each group; however day 10 represents n=9 observations in the PC group due to one mortality. Median titer by day (B) represents the proportion of chickens shedding H6N2 virus at that day (C). For each day from left to right: Cocktail; Scramble; PC.

### DETAILED DESCRIPTION OF THE PREFERRED EMBODIMENT

Developing a powerful anti-influenza technology is a critical step to effectively manage and control the spread of AIV in poultry to minimize financial losses and risks for transmission to other susceptible species, including humans. Current vaccination strategies for birds are limited as they do not confer complete immunity, are reliant on a healthy immune system, are susceptible to viral antigenic evolution, require individual handling of every bird in a large scale commercial poultry operation, have a limited shelf-life, and antibody protection following vaccination takes several weeks to acquire. Because of these challenges, AIV vaccination within the US is rarely favored for prophylactic use in poultry. If an emergency vaccination program were adopted, it would offer little protection if administered during an outbreak. Furthermore, growing evidence for the emergence of drug resistance AIV variants poses a risk for the use of drug therapies, such as amantadine, rimantadine and oseltamivir. These limitations indicate a real need to develop a prophylactic that can not only protect against different subtypes and drug resistant strains of virus, but would provide transient protection should an outbreak occur and vaccination is not feasible or effective in a short period of time.

RNA interference (RNAi) using siRNAs has been explored for the development alternative antivirals to control diseases in humans and livestock species using siRNAs (Lyall et al 2011, Long et al 2010, Chen et al 2009). The delivery of RNAi-mediating agents has been an obstacle to its clinical application (Li and Shen 2009). Since siRNAs are unable to cross cell membranes independently, they require a delivery vehicle such as genetically engineered viruses and synthetic carriers (Aigner 2009, Li et al 2006, Ge et al 2004). These viral and synthetic siRNA vehicles pose serious limitations and concerns for clinical efficacy (Ge et al 2004). Viral vectors can elicit strong immune responses causing cell death (Davidson and McCray 2011, Ge et el 2004). Controlling the dose of RNAi agents with viral vectors is difficult and can result in saturation of the RNAi/microRNA systems resulting in hepatotoxicity (Beer et al 2010, Hacein-Bey-Abina et al 2008, Grimm et al 2006). Viral vectors can integrate into the host's genome and lead to tumorigenesis (Davidson and McCray 2011, Beer et al 2010, Hacein-Bey-Abina et al 2008, Ge et al 2004). In fact, recent studies warn of off-target effects from short hairpin RNA (shRNA) viral vectors resulting in cell death and organ problems in transgenic animals (Grimm et al 2006). Finally, synthetic RNA vehicles have low delivery efficiencies and require higher doses, which is not only cost prohibitive, but often toxic (Ge et al 2004). Consequently, it is imperative to focus on better delivery mechanisms for RNAi-based antivirals, specifically against AIV in poultry. A safe and effective siRNA delivery vehicle that would specifically target the lung and respiratory tissues, the main sites of AIV infection and replication, would represent a particularly promising approach. This type of system would improve the specificity of delivery for a more effective anti-influenza prophylactic, while minimizing siRNA losses due to systemic administration and reducing siRNA related toxicity.

Accordingly, a transkingdom RNAi (tkRNAi) delivery platform was developed to provide for the inhibition of AIV in chicken epithelial cells. TkRNAi uses nonpathogenic bacteria to generate and deliver silencing RNAs to mucosal epithelial tissues, and proves to be a promising delivery approach for preventing influenza via administration to the upper airways in chickens. These bacteria are genetically engineered to produce shRNA specific for an mRNA target, invade mucosal epithelial cells, and release their shRNA payload into the host cells' cytoplasm. It is thought that once released into the cytoplasm these shRNA are processed into siRNA and subsequently silence complementary mRNA targets, thereby triggering RNAi (Buttaro and Fruehauf 2010, Xiang et al 2006). The tkRNAi system used herein is comprised of *E*. *coli* bacteria that have been transformed with a special shRNA generating plasmid (pMBV43). These pMBV43 plasmids and carrier *E. coli* bacteria are characterized by several components. These include an shRNA expression cassette under the control of the T7 RNA polymerase promoter, and terminator for successful release and generation of siRNAs in the cytoplasm of influenza susceptible respiratory epithelial cells. The introduction of an invasion gene (*inv*) from *Yersinia pseudotuberculosis* provides for the expression of invasin protein on the *E*. *coli* surface. Invasin interacts with β1 integrin receptors present on the surface of mucosal epithelial cells leading to uptake of the carrier bacteria into endosomes of the target epithelial cells. Once the *E*. *coli* are taken up into the host cell, they need to escape the host vacuole for release in the cytosol. The bacteria are encoded to express Listerolysin O (LLO) from the *hlyA* gene, a pore-forming toxin from *Listeria monocytogenes* (Xiang et al 2006, Mathew et al 2003, Radford et al 2002, Grillot-Courvalin et al 1998). Due to the low pH environment of the host lysosome and a lack of nutrients, the bacteria are lysed inside the endosome, subsequently releasing this bacterial toxin, which leads to the rupture of the endosomal membrane (Nguyen and Fruehauf 2009). Now in the cytoplasm, the released shRNA are processed into siRNA by Dicer, incorporated into the RNA-induced Silencing Complex (RISC), and trigger the RNAi pathway to silence the genes being targeted for knockdown. Using tkRNAi, we developed a novel RNAi antiviral capable of generating and delivering siRNAs targeting the NP and PA AIV genes. This novel approach was applied to an avian tissue model, whose development is also disclosed herein, to demonstrate *in vitro* proof of concept for using anti-AIV vectors to inhibit avian influenza replication and shedding.

Economic incentives to vaccinate poultry against AIV are low and often owed to several limitations of the vaccine. These limitations and lack of incentive pose significant hurdles for effectively controlling AIV outbreaks in poultry. Developing a new anti-influenza technology is a critical step towards effectively managing and controlling the spread of this disease in poultry, minimizing financial losses, and reducing the risk for transmission to other animals, including humans. Applying RNAi methodologies to develop an alternative antiviral against AIV is a promising option. However, the delivery of RNAi-mediating agents remains an obstacle to harnessing its clinical application. Transkingdom RNAi (tkRNAi) uses nonpathogenic bacteria to generate and deliver siRNAs to target tissues, and could be the key to attaining clinical application of an RNAi approach. TkRNAi vectors (anti-AIV vectors) were constructed to generate siRNA targeting the viral genes, NP and PA, and the protective efficacy of these vectors was evaluated in the established avian tissue model.

Vector uptake and invasion was first verified in chicken LMH cells treated with vectors tagged with a red fluorescent protein. Next, cells were treated with the anti-AIV vectors and infected with two different LPAI subtypes, H8N4 and H6N2. Multivariable linear regression analysis, controlling for day, revealed significant differences in adjusted mean shedding titers between samples treated with the anti-AIV vectors and those untreated (p<0.05). Vector cocktails targeting both NP and PA genes provided up to a 10,960-fold reduction in shedding titers in chicken LMH cell supernatants (p<0.05). This work demonstrates *in vitro* proof of concept for using these novel anti-AIV vectors to inhibit AIV in the established avian tissue model. This work represents the first such instance for using the tkRNAi system in an avian model and to inhibit influenza replication.

Because the NP and PA proteins both play a critical role in AIV transcription and replication, they represent a prime target for the inhibition of virus replication by siRNA. Designing siRNAs that can remain potent through antigenic drifts and shifts requires targeting regions within a gene that are conserved among different subtypes and strains of AIV. Due to their role in viral transcription and replication and as a way of maintaining optimal viral fitness, NP and PA each contain a stretch of conserved nucleotides found in most Type A influenza viruses. The specific role these short sequences play in viral replication has yet to be determined. However, these short conserved sequences are found in chicken, human, canine, equine, and swine influenza genomes. Unlike the NP and PA genes, the HA and NA segments contain no stretch of 21 conserved nucleotides for siRNA design. The reliance on these conserved regions for successful transcription and replication render these short sequences in the NP and PA genes prime targets for siRNA design.

Nearly all research investigating the application of siRNAs is assessed in mammalian cells, and studies using avian models are lacking. The use of mammalian cells is a significant limitation for a couple of reasons. First, as the majority of RNAi design tools were developed using mammalian systems, these tools might design siRNAs that are less effective when introduced into avian cell cultures, leading to lower RNAi efficiencies. Second, AIV is a disease of avian species. As such, it is imperative to use a relevant avian tissue model to determine if RNAi is a valid approach to preventing AIV replication in poultry. The viruses typically selected for previous RNAi transfection and viral infection studies are lab-adapted viruses, isolated from humans and other mammals. If we seek to determine if RNAi is a valid approach to preventing AIV replication in poultry, it is critical to conduct these studies using naturally occurring avian influenza viruses commonly isolated from poultry.

AIV is recognized as a National Institute of Allergy and Infectious Diseases bio-defense category C priority pathogen, an economically important disease of poultry, and a major public health threat because viruses originating from animals have the potential to cause the next human pandemic (Carver and Krushinskie 2006, Webster 1997). AIV affects many species of food production birds, including chickens, turkeys, ducks, quails, and guinea fowl. Throughout the literature, there are countless descriptions of significant global outbreaks of LPAI and HPAI in domestic poultry. These outbreaks are of concern, not only because of the degree of virulence observed in poultry resulting in severe economic consequences, but due to the ability to transmit to mammalian species. Highly pathogenic H5N1 avian strains are currently circulating in multiple countries. HPAI outbreaks in poultry have led to the culling of millions of animals and the net loss of billions of dollars. Since 2003, H5N1 strains have resulted in 648 human cases, of which 384 were fatal (2013: WHO Influenza at the human animal interface). Shockingly, human infection with the LPAI (H7N9) virus has now been reported, with approximately 150 cases of which nearly 33% have resulted in death (Centers for Disease Control and Prevention 2014).

A major risk factor for AIV transmission to humans is direct contact and handling of domestic birds (Vong et al 2009, Areechokchai et al 2006, Dinh et al 2006, Wang et al 2006, Bridges el al 2002, Mounts et al 1999), so the majority of human cases have occurred when close proximity between humans and livestock leads to transmission. As the number of circulating viruses increases in domestic poultry, so does the risk for transmission to humans and the potential to reassort into a form that is more transmissible among humans (Gatherer 2010). Therefore, with respect to reducing the risk to humans, AIV prevention in domestic poultry must be a major focus. The emergence of a novel, transmissible AIV in a population that has little or no immunity, would cause a global pandemic for which no vaccine is available. These strains are of particular concern because they are resistant to the most cost-effective antiviral drugs, amantadine and rimantadine (He et al 2008, Lee et al 2008, Pinto and Lamb 1995, Belshe et al 1988).

Just as it is critical to have early detection protocols and warning systems in place as means to control and manage AIV in poultry, effective prevention measures must be available to prepare for potential outbreaks. Vaccinating domestic poultry may help to control HPAI viruses and reduce economic losses and zoonotic transmission, however vaccination is not a common practice on poultry farms because it is not seen as a viable option. Furthermore, if vaccination were adopted in an emergency situation, it would likely occur after an outbreak has already been identified. Vaccine protection takes two to three weeks to acquire (Kim et al 2009), and often requires repeated doses to induce full protection (Poetri et al 2011). Therefore, vaccination during an outbreak might do little to control the chain of transmission, especially in densely populated poultry areas. Developing a powerful anti-influenza technology for poultry is a critical step to effectively manage and control the spread of this disease worldwide.

The work previously described investigated the inhibition of AIV in chicken epithelial cells using the RNAi delivery platform, tkRNAi. This tkRNAi system uses engineered E. coli to produce short hairpin RNA against the NP and PA viral genes and successfully delivered the shRNA intracellularly. Within twenty-four hours of administration, these vectors lead to significant reductions in AIV replication in vitro. These proceedings demonstrated the transient antiviral potential of these novel anti-AIV vectors in our avian tissue model. In continuing pursuit of our long-term goal to develop this anti-influenza technology into a prophylactic that would contribute to the development of a more effective and robust control method for AIV in poultry, the aim of the present study is to evaluate the intranasal administration and the protective efficacy of these antiviral vectors in experimentally challenged chickens.

Vaccine efficacy is evaluated on the basis of clinical protection (reduced morbidity and mortality) and through the detection of virus shedding after challenge. An additional goal of AIV vaccination in poultry is to stimulate an immune response and prevent the individual animal from getting infected. This prophylactic is not engineered to prevent infection in the individual animal. This novel technology's mode of action is inhibiting viral replication, thereby reducing the shedding of infectious virus following infection.

Theoretically, if a chicken is shedding lower amounts of virus, the viral load is reduced and the animal is more likely to be clinically protected. Therefore, to provide initial efficacy for these anti-AIV vectors, the prophylactic ability of these vectors to reduce morbidity, mortality, and viral shedding in experimentally challenged chickens was evaluated. If this prophylactic can inhibit the proportion of birds shedding and shedding titers in the individual bird, the risk for transmission will be better controlled among populations of birds. Therefore, in addition to monitoring the proportion of shedding birds and the shedding titers, the potential for transmission has been tested using sentinel birds housed among AIV challenged birds that received the anti-AIV vectors (see Example 3, below).

Poultry treated with the anti-AIV vectors would not develop antibodies against AIV. This is typically a limitation associated with vaccination practices because the vaccine stimulates the production of antibodies against the virus. This creates problems related to trade barriers, making it difficult to distinguish antibody reactivity due to vaccination verses natural AIV infection. If an effective conventional vaccine is available, it often cannot be used. A country cannot risk losing AIV disease-free status, which is often the case when positive serological tests due to vaccination interfere with disease surveillance. Unlike vaccinated poultry, those treated with the anti-AIV vectors would not test positive for AIV antibodies, and would not require further testing using the Differentiate Infected from Vaccinated Assay (DIVA). In this way, trade barriers associated with the reactivity of vaccinated animals and additional screening tests for AIV would be avoided.

The poultry industry will not adopt a commercial vaccine or any other prophylactic without demonstrating that its use is a cost-effective countermeasure to outbreaks of AIV. This is especially critical in low-density poultry areas where the risk for an outbreak is perceived as negligible. Factors that affect the cost of vaccination include shelf life or sustainability of the vaccine, method of administration, ease at which it can be generated, and accessibility. Generally, the benefit must be higher than the cost of vaccination or treatment to be viewed as a good investment.

One of the limitations associated with other RNAi therapeutic approaches is the cost associated with the commercial manufacturing of the silencing RNA. An obvious advantage of the proposed technology is the ability of the bacteria themselves to produce the shRNA for cleavage into the subsequent siRNA sequences. This characteristic eliminates expensive siRNA manufacturing and high production costs (Xiang et al 2009, Keates et al 2008, Xiang et al 2006, Gardlik et al 2005). In areas where vaccines are considered necessary, countries will stockpile millions of doses of vaccine each year for emergency preparedness. Many times these vaccines become obsolete when they are found to be distant to the field virus. It is simple and fast to generate large quantities of bacteria. Therefore, unlike a vaccine that can take months to formulate and even longer to acquire in sufficient quantities, these vectors could be readily generated in massive quantities from a small stock. If properly stored, these bacterial vectors could have an indefinite shelf life. This is yet another major advantage over current vaccines.

Vaccination and other RNAi approaches have their share of hurdles related to administration and targeted delivery. Most AIV vaccines must be administered subcutaneously making them cumbersome and inefficient to administer. These anti-AIV vectors allow for targeted respiratory delivery and specific invasion into mucosal epithelial cells expressing β1-integrins. Unlike other tissues and organs, respiratory tissues including the lungs are incredibly accessible, making this anti-AIV technology ideal for preventing respiratory diseases like influenza. Chickens have a gland located medial to the eye called the Harderian gland, and this gland is thought to be critical for developing a local immune response in the upper respiratory tract (Wight et al 1971). For this reason, eyedrop vaccinations are believed to stimulate a mucosal immune reaction via the Harderian gland and the same can be said if these vectors were administered into the eye. The anti-AIV vectors could be easily amendable for administering intranasally, orally, via eyedrop, or as a sprayed aerosol suitable for inhalation or ocular delivery.

These vectors would represent a cost effective, sustainable product readily available for large-scale application on a commercial poultry operation. The versatility of delivery and the ease at which it could be amendable for large-scale application gives this technology an advantage over current vaccines and equates to a system that provides efficient delivery, and localization to lung tissues. These qualities make this technology especially advantageous during an outbreak situation to reduce shedding and transmission of virus between poultry populations.

Efficacy is an essential characteristic of an ideal AIV vaccine or alternative prophylactic for poultry. The term efficacious is used to determine if a vaccine or alternative prophylactic is protective in standardized experimental challenge studies, as measured by preventing mortality, morbidity, shedding, and transmission of AIV among poultry. The industry standards for vaccine efficacy with HPAI are reduced morbidity and mortality. Very little morbidity and mortality are observed in LPAI experimentally challenged SPF chickens, making it difficult to determine vaccine efficacy for LPAI isolates. Quantifying oropharyngeal titers from chickens is routinely how efficacy is determined for vaccines against LPAI isolates. Because vaccines currently available for poultry do not provide sterilizing immunity, an effective control tool should be reducing virus shedding to the greatest extent possible (Sylte and Suarez 2012). The results from these initial pilot studies indicate that anti-AIV vector treatment reduces virus replication and shedding titers to levels considered clinically relevant. However, additional studies need to be conducted to further demonstrate the protective nature of this technology.

Vaccination for AIV in poultry is more difficult than vaccination used against most other disease causing viruses. This is owed to large antigenic variations that require prior knowledge of circulating subtypes, reducing vaccine efficacy. Low vaccine efficacy and high cost to benefit ratios usually offset any proposed vaccination policies. When AIV is detected in poultry, generally a 'stamping out' or culling policy is used in the efforts to eradicate the disease. Assuming efficacy, potency, and environmental safety is demonstrated in a future challenge studies and randomized field trials, I envision this prophylactic technology could be a solution to preemptive culling of poultry in high-risk areas. However, even when vaccination is selected as an emergency tool during outbreaks, it is often in conjunction with other control methods, especially if rapid culling is difficult. Previous experiences have indicated that in order to be successful at controlling and ultimately in eradicating AIV, vaccination programs must be part of a wider control strategy (Capua and Marangon 2006). This includes strict biosecurity measures, surveillance, culling, and possibly implementing the use of this prophylactic technology.

Another issue related to efficacy is the time interval necessary to obtain protective immunity. It requires a minimum of 7 to 10 days to stimulate an initial immune response following vaccination, and another 2 weeks to generate protective antibody levels. This implies that even if the decision-making process is fast-tracked and an appropriate vaccine is available for immediate use, vaccination might not be efficacious if applied during an active outbreak in poultry. A key benefit this anti-AIV vector technology would have over the vaccine is its ability to provide transient protection. This characteristic would be incredibly advantageous during an outbreak situation.

### Example 1 -Design of siRNAs and use of viral specific siRNAs to inhibit avian influenza in an avian tissue model.

Avian influenza virus (AIV) represents one of the most significant economic threats to poultry worldwide. Vaccines for AIV are limited, highlighting the need to consider new prophylactic strategies that can protect poultry against outbreaks. An appropriate avian tissue transfection and AIV infection model was developed. This avian model was used to demonstrate the antiviral potential of small interfering RNA ("siRNA") targeting two key AIV genes required for viral replication; NP and PA. Chicken LMH cells were transfected with siRNAs targeting NP and PA mRNA and cells were infected with two different LPAI subtypes, H8N4 and H6N2. Multivariable linear regression analysis, controlling for day, revealed significant differences in adjusted mean shedding titers between samples treated with siRNA and those untreated (p<0.05). Individual siRNAs and tested siRNA cocktails led to a decrease of up to 100 fold in shedding titers in chicken LMH cell supernatants (p < 0.05). This work demonstrates *in vitro* proof of concept for using viral specific siRNAs to inhibit avian influenza replication in the established avian tissue model.

### MATERIALS AND METHODS

Cell cultures - Chicken hepatocellular carcinoma epithelial cells (LMH cells, ATCC CRL-2117) were chosen as the model avian cell line. These cells are commercially available, specifically intended for transfection studies, and as epithelial hepatocytes, they represent an appropriate avian tissue for type A influenza infection (Swayne and Pantin-Jackwood 2006, Shinya et al 1995). LMH cells were grown in Waymouth's MB 752/1 medium (Life Technologies), with 10% fetal bovine serum (FBS, Life Technologies), 2 mM L-Glutamine (Life Technologies), 100 Units/ml penicillin and 1mg/ml streptomycin (Life Technologies) at 37°C in the presence of 7% CO₂. All culture vessels used to propagate the LMH cells were precoated with 0.1% gelatin (Millipore). Madin-Darby canine kidney (MDCK) cells were used to determine viral titers and to quantify infectious viral particles following all transfection and infection assays conduced in LMH cells. MDCK cells were grown in minimum essential medium with Earle's balanced salts (MEM/EBSS), 2 mM L-Glutamine, 10% fetal bovine serum (FBS), 0.5% sodium pyruvate (100 mM solution, Life Technologies), 0.5% MEM non-essential amino acids (NEAA, 100x solution, Life Technologies), and 100 Units/ml penicillin and 1mg/ml streptomycin (Life Technologies) at 37°C in the presence of 7% CO₂.
Viruses - The following LPAI avian influenza virus strains were propagated in LMH cells: A/Chicken/Texas/473-2/10 (H6N2), A/Chicken/Texas/55116-2/11 (H4N8), and LPAI subtype H8N4 isolated from a turkey outbreak in Colorado, 2005 (subtype was previously determined by hemagglutination-inhibition and neuraminidase-inhibition panels). Virus titers were measured by 50% tissue culture infectious dose (TCID50) assay in MDCK cells. H6N2 and H8N4 LPAI strains were chosen for the study because they replicated to high titers in LMH cells with strong cytopathic effect (CPE) at 48 hours post infection (hpi) and are suitable for TCID₅₀ assays using MDCK cells. Both viruses were propagated in LMH cells and the infectious viral supernatants were collected, centrifuged to remove cell debris, and stored at -80°C. A working stock titer was measured by TCID₅₀ and optimal multiplicity of infection (MOI) was determined.
siRNAs - RNA oligonucleotides were synthesized, desalted, and duplexed prior to shipment (Dharmacon, Thermo Scientific). Sense and antisense RNA sequences for NP (NP-siRNA) and PA (PA-siRNA) were previously published (Ge et al 2003). The mRNA target sequence for each siRNA are as follows: NP: 5'- GGA TCT TAT TTC TTC GGA G - 3' (SEQ ID NO. 1) and PA: 5' -GCA ATT GAG GAG TGC CTG A - 3' (SEQ ID NO. 2). BLAST was used to verify each siRNA sense strand sequence was 100% complementary to the NP and PA viral gene sequences associated with H6N2 and H8N4. Additionally, all siRNA anti-sense seed region sequences were screened for high homology to chicken sequences to decrease the possibility of off-target silencing.
Transfection efficiency - Several transfection reagents were tested for visual signs of CPE independent of siRNA. These include Lipofectamine® 2000 (Life Technologies), Lipofectamine® RNAiMax (Life Technologies), FuGene® 6 (Promega Corporation) and XtremeGene (Roche Diagnostics). Transfection efficiencies were determined in LMH cells using BLOCK-iT™ Alexa Fluor® Red Fluorescent dsRNA Control (Life Technologies). Alexa Fluor®-positive cells were counted in eight random ocular fields at 40X using the LSM 510 Zeiss confocal fluorescent microscope. Fluorescent microscopy was used to select the final transfection reagent, determine the optimal concentration range of siRNA and transfection reagent, and the optimal siRNA incubation period. The transfection efficiency (α = 0.05) was determined after 24 h incubation.
Transfection of siRNA into chicken cells - LMH cells were seeded one day prior to transfection in 24-well plates to allow cell monolayers to reach 80% confluency. Cells were transfected with NP-siRNA, PA-siRNA and both NP/PA (cocktail-siRNA) (32nM individually or 64nM total in a cocktail format) using Lipofectamine® 2000 according to the manufacturer's instruction. Briefly, Lipofectamine® 2000 was diluted 1:50 in Opti-MEM medium (Life Technologies). Each test siRNA and mock-siRNA (scrambled negative siRNA with no complementarity to any known chicken gene) was subsequently diluted in the Lipofectamine® 2000 - Opti-MEM mixture and allowed to incubate at room temperature for 15 minutes. The LMH cells were prepared for transfection by removing the complete growth medium and washing each well with Opti-Mem medium only. The wash was replaced with 687 µl/well transfection medium (Opti-MEM, 10% FBS, 2 mM L-Glutamine) and 63 µl of siRNA-Lipofectamine® 2000 complex was added. All control or treated cells were transfected in triplicate wells.
Virus infection - Twenty four hours post transfection the LMH cells were prepared for infection by removing the transfection medium and washing each well with inoculation medium (IM) containing 0.25 µg/ml TPCK treated trypsin (Sigma-Aldrich), 3% bovine serum albumin (BSA, Gemini Bio-Products), 2 mM L-glutamine, 100 U/ml penicillin and 1mg/ml streptomycin. The wash was removed and a total of 250 µl of IM containing either H8N4 or H6N2 virus at an MOI of 0.01 or 0.001 was added to each appropriate well. Plates were incubated on a rocking platform at 37°C in the presence of 7% CO₂ for 1 hour before removing the virus and replacing with 750 µl/well of fresh IM. Forty-eight hpi cell culture supernatants were harvested, centrifuged to remove cell debris, and frozen at -80°C. Each experiment included a positive control (LPAI infected only), negative control (untreated LMH cells) and mock-transfection control (mock-siRNA and LPAI infected) tested in triplicate wells.
Evaluation of infectious virus titer - LMH cell culture supernatants were quantitated by titration on MDCK cells using CPE as the indicator of viral presence, and titer was expressed as the 50% tissue culture infectious dose/ml (TCID50/ml). Briefly, MDCK cells were seeded into 96 well plates. For each culture supernatant, ten-fold serial dilutions from 1:10 to 1:108 were made in MEM/EBSS, 2 mM L-Glutamine, 3% BSA, 0.5% sodium pyruvate, 0.5% MEM-NEAA, 100 U/ml penicillin and 1 mg/ml streptomycin, containing 1 µg/ml TPCK treated trypsin (Worthington Biochemical Corporation). When cell monolayers were 80-90% confluent, growth medium was removed and the cells were inoculated with 100 µl/well of virus suspension in triplicate. At 48 hpi cells were incubated with crystal violet stain (0.1% crystal violet and 2.5% PBS buffered formalin in phosphate buffered saline (PBS)) and wells with CPE were scored as positive for virus growth. TCID50/mL was calculated by the Reed and Muench mathematical technique (Reed and Muench 1938).
Statistical Analysis - All experiments were repeated a minimum of three times, on different days. Using a statistical significance level of 0.05, the Wilcoxon rank-sum test was performed to compare median viral titers between siRNA transfected and positive control (PC) samples performed on the same day. To determine if adjusted mean LogTCID₅₀/mL values between siRNA transfected and PC samples were statistically different (p < 0.05), data was analyzed using multivariable linear regression, controlling for day. Statistical analyses were performed using the computing software Stata 10 (Statcorp LP).

### RESULTS

Design of effective siRNAs - The wide range of genetic variation among different strains of avian influenza viruses is largely responsible for viral evasion following nonspecific vaccination. Likewise, this genetic variability makes it difficult to design siRNAs that can remain effective against multiple AIVs. As little as one nucleotide difference between the mRNA target site and the siRNA antisense seed region can completely abolish any RNAi antiviral activity. Therefore, it is important to design siRNAs targeting highly conserved sequences observed across many different strains, including HPAI viruses. BLAST was used to search for sequence homology in type A influenza viruses as well as off-target matches between the NP and PA siRNA sequences and chicken sequences. This screening revealed neither siRNA had high homology to any known chicken sequences, specifically between the siRNA seed region and host mRNA. The NP and PA siRNA sequences both aligned against the H8N4 and H6N2 NP and PA sequences with 100% homology. Using the nucleotide collection (nr/nt) database for both 19 nt siRNA sequences, the BLAST search reveals 100% query cover in over 10,000 type A influenza viruses, including those isolated from swine, wild birds, poultry, equine, canine, and humans. These query matches include pathogenic H5, H9, and H7 subtypes isolated as recently as 2013 from national and international outbreaks.

siRNA antiviral activity in chicken cells - The transfection procedure was optimized to achieve up to 81% transfection rate in LMH cells using Lipofectamine® 2000 and siRNA concentration between 32nM and 64nM. This transfection procedure and range of siRNA concentration allowed for adequate transfection, without inducing CPE. Prior to infection with H6N2 or H8N4 virus at MOI 0.01 and 0.001, NP and PA-siRNA were transfected into LMH cells and culture supernatants were collected for TCID₅₀ analysis. Lack of significant differences in virus titer between mock controls and PCs (data not shown) indicate that siRNA transfection did not affect virus production.

As expected, the antiviral activity of each siRNA transfection varies by day, virus, and MOI used for infection (FIG. 1). However, significant antiviral activity is observed in NP, PA, or cocktail-siRNA samples from both H6N2 and H8N4 infections. On each experimental day, at least one siRNA transfection significantly reduced median virus titers in both viruses and at both MOIs, compared to the corresponding PC samples. On at least one experimental day, the cocktail-siRNA significantly reduced median virus titers in both viruses and at both MOIs, except H8N4 at MOI 0.001. Despite a lack of significant differences likely associated with a small sample size, 70%, 50%, and 100% of all NP-siRNA, PA-siRNA, and cocktail-siRNA transfections resulted in lower median viral titers compared to their appropriate untreated PCs, respectively.

To compare siRNA transfected to untreated PC samples across all experimental days, multivariable linear regression (MLR) analysis was used, controlling for day. MLR results reveal significant differences in adjusted mean viral titers between siRNA transfected and untreated PC samples (Table 1). Significant differences are observed in both NP-siRNA and cocktail-siRNA samples from H8N4 MOI 0.01 and H6N2 MOI 0.001 infected cells. The log₁₀ reduction in infectious virus between siRNA transfected and untreated samples were calculated. With the exception of PA-siRNA transfection from H8N4 MOI 0.001 and H6N2 MOI 0.01 samples, all siRNA transfections resulted in at least 0.3 log₁₀ reduction (2.1 fold reduction). Most notable were H8N4/MOI 0.01 titers after cocktail-siRNA transfection, H8N4/MOI 0.001 titers after NP-siRNA transfection, and H6N2/MOI 0.001 titers following cocktail and NP-siRNA transfections, resulting in a 1.3 (20-fold), 1.0 (10-fold), and 1.7 (50-fold), 2.0 log₁₀ reduction (100-fold), respectively.

**Table 1 - SiRNA protection in chicken epithelial cells as measured by log and fold-reductions in shedding titers.**

| Sample | Adjusted Mean^{a} | 95% Cl | Adjusted Mean (untreated) | 95% Cl (untreated) | P-value^{b} | Log reduction^{c} | Fold reduction^{d} |
|---|---|---|---|---|---|---|---|
| H8N4 LPAI virus MOI 0.01 | | | | | | | |
| NP-siRNA | 4.1 | (3.4, 4.8) | 5.0 | (4.3, 5.7) | 0.01 0 | 0.9 | 7.9 |
| PA-siRNA | 5.5 | (4.5, 6.5) | 5.8 | (4.8, 6.8) | 0.54 7 | 0.3 | 2.0 |
| Cocktail-siRNA | 3.7 | (3.1, 4.3) | 5.0 | (4.4, 5.6) | < 0.00 1 | 1.3 | 20.0 |

| H8N4 LPAI virus MOI 0.001 | | | | | | | |
|---|---|---|---|---|---|---|---|
| NP-siRNA | 7.5 | (6.4, 8.7) | 8.5 | (7.6, 9.4) | 0.08 8 | 1.0 | 10.0 |
| PA-siRNA | 8.6 | (7.3, 10.0) | 8.5 | (7.5, 9.6) | 0.89 2 | -0.1 | 0.8 |
| Cocktail-siRNA | 8.3 | (7.1, 9.4) | 9.0 | (8.1, 9.9) | 0.19 0 | 0.7 | 5.0 |

| H6N2 LPAI virus MOI 0.01 | | | | | | | |
|---|---|---|---|---|---|---|---|
| NP-siRNA | 6.3 | (5.5, 7.2) | 6.6 | (5.7, 7.5) | 0.44 3 | 0.3 | 2.1 |
| PA-siRNA | 7.5 | (6.7, 8.2) | 6.9 | (6.1, 7.7) | 0.10 9 | -0.6 | 0.3 |
| Cocktail-siRNA | 5.8 | (4.9, 6.6) | 6.3 | (5.5, 7.1) | 0.18 8 | 0.5 | 3.2 |

| H6N2 LPAI virus MOI 0.001 | | | | | | | |
|---|---|---|---|---|---|---|---|
| NP-siRNA | 8.4 | (7.2, 9.7) | 10.4 | (9.5, 11.4) | 0.00 4 | 2.0 | 100.0 |
| PA-siRNA | 10.0 | (8.4, 11.7) | 10.4 | (9.2, 11.7) | 0.61 5 | 0.4 | 2.5 |
| Cocktail-siRNA | 8.7 | (7.4, 10.0) | 10.4 | (9.4, 11.5) | 0.01 2 | 1.7 | 50.1 |
| Results are from multivariable linear regression, controlling for day. ^{a}Expressed as adjusted mean LogTCID₅₀/mL. ^{b} Comparing adjusted mean LogTCID₅₀/mL from treated to untreated PC samples (p<005). ^{c} Logreduction in mean infectious titer compared to untreated control. ^{d} Fold reduction in geometric mean compared to untreated control. | | | | | | | |

This example demonstrates *in vitro* proof of concept for using viral specific siRNAs to inhibit avian influenza in an avian tissue model. Chicken LMH cell transfection with siRNAs targeting NP and PA mRNA significantly reduced infectious titers following infection with two different AIV subtypes, H8N4 and H6N2. Both of these viruses are not lab adapted LPAI viruses. Both were isolated from past outbreaks in poultry, occurring in the United States. As such, they are appropriate and ideal viruses to test this siRNA antiviral approach.

Even when a significant difference between siRNA transfection and untreated PC samples was not observed, log₁₀ reductions in infectious viral titers were observed in all siRNA samples, except two PA-siRNA transfection sets. The most potent inhibition of infectious viral titers occurred after LMH cells were transfected with either NP-siRNA or cocktail-siRNA, resulting in log₁₀ reductions ranging from 0.5 - 2.0 (3.2 - 100 fold reduction).

This work provides a basis for defining measures eliciting sufficient siRNA protection that prevents virus shedding and subsequent disease transmission during an AIV outbreak in poultry. Additionally, influenza viruses mutate quickly, often rendering vaccines ineffective. Designing RNAi constructs targeting these conserved mRNA sites could allow for more efficient RNAi silencing, but moreover, targeting multiple conserved sites using these siRNA cocktails could further prevent RNAi escape by mutation.

RNAi is a tool that can help researchers achieve what has not yet been possible and provide an opportunity for a new anti-influenza development strategy. This study was successful at developing an appropriate *in vitro* avian model to validate this RNAi anti-influenza approach. This work showed that siRNA-mediated knockdown targeting both the viral NP and PA genes, in a cocktail format, inhibits AIV replication *in vitro.* However, despite the potential these siRNA have to inhibit infectious virus shedding in this avian model, these RNAi-mediating agents require a better mechanism of delivery to be deemed clinically applicable. The efficiency of delivery associated with using a synthetic transfection vehicle, such as Lipofectamine® 2000, is limited for several reasons. First, low delivery efficiencies require higher doses, which are not only cost prohibitive, but often toxic (Ge et al 2004). Second, these transfection reagents only achieve systemic delivery, not targeted delivery. This again requires higher doses to sufficiently transfect a specific tissue. Developing an RNAi antiviral technology that would allow for intranasal delivery would be uniquely promising for the prevention of AIV. Consequently, subsequent work focused on designing a better delivery mechanism for this RNAi based antiviral, specifically for the prevention of AIV in poultry. This work is presented below in Example 2.

### Example 2 - Inhibiting Avian Influenza Replication in a Chicken Cell Model Using a Unique RNAi Delivery Technology

Economic incentives to vaccinate poultry against AIV are low and often owed to several limitations of the vaccine. These limitations and lack of incentive pose significant hurdles for effectively controlling AIV outbreaks in poultry. Developing a new anti-influenza technology is a critical step towards effectively managing and controlling the spread of this disease in poultry, minimizing financial losses, and reducing the risk for transmission to other animals, including humans. Applying RNAi methodologies to develop an alternative antiviral against AIV is one possibility. However, the delivery of RNAi-mediating agents remains an obstacle to harnessing its clinical application. Transkingdom RNAi (tkRNAi) uses nonpathogenic bacteria to generate and deliver siRNAs to target tissues, and could be the key to attaining clinical application of an RNAi approach. TkRNAi vectors (anti-AIV vectors) were constructed to generate siRNA targeting the viral genes, NP or PA, and the protective efficacy of these vectors was evaluated in the established avian tissue model. Combinations of vectors ("cocktail") were also employed to investigate the additive effect of targeting multiple AIV mRNA. Vector uptake and invasion was first verified in chicken LMH cells treated with vectors tagged with a red fluorescent protein. Next, cells were treated with the anti-AIV vectors and infected with two different LPAI subtypes, H8N4 and H6N2. Multivariable linear regression analysis, controlling for day, revealed significant differences in adjusted mean shedding titers between samples treated with the anti-AIV vectors and those untreated (p<0.05). Vector cocktails targeting both NP and PA genes provided up to a 10,960-fold reduction in shedding titers in chicken LMH cell supernatants (p<0.05). This work demonstrates *in vitro* proof of concept for using these novel anti-AIV vectors to inhibit AIV in the established avian tissue model. This work represents the first such instance for using the tkRNAi system in an avian model and to inhibit influenza replication.

### MATERIALS AND METHODS

Cell cultures - Based on previous work in Example 1, chicken hepatocellular carcinoma epithelial (LMH) cells and Madin-Darby canine kidney (MDCK) cells were used for all invasion and infection, and viral titer assays, respectively. Cells were maintained in appropriate growth medium, as previously described, with slight modification. To minimize host complement inactivation of the anti-AIV vectors during the invasion step, LMH cells were continuously grown in medium containing heat inactivated FBS (Life Technologies). This was to avoid possible activation of the alternative complement pathway in response to the presence of bacteria, leading to inactivation of the bacterial vectors prior to intracellular uptake.

Viruses - The following LPAI avian influenza virus strains were chosen for use in all invasion and infection studies: A/Chicken/Texas/473-2/10 (H6N2) and LPAI subtype H8N4 isolated from a turkey outbreak in Colorado, 2005. Viral titers for each LPAI were measured by TCID₅₀ assay. Both viruses replicate to high titers in LMH cells with CPE at 48 hours post infection (hpi). As previously described, stocks were generated and optimal MOls chosen.

Beta-1 Integrin validation in chicken cells - Because avian tissues are a novel target for tkRNAi, the presence of β(1) integrin receptors on the surface of LMH cells under normal conditions (uninfected) and post AIV infection was important to validate. Total RNA was extracted (E.Z.N.A.® Total RNA Kit I, Omega Bio-Tek) from LMH cell cultures that were uninfected (normal growth conditions) and from cultures at 6 and 24 hours post infection with H8N4 virus. First strand cDNA was synthesized from 0.75 µg of total RNA using Oligo(dT) Primer (Promega) and the cDNA synthesis was completed using 4mM dNTPs and the AffinityScript Multiple Temperature Reverse Transcriptase kit (Stratagene), according to the manufacturer's recommendations. The housekeeping gene, β-actin, was used as an internal control for β(1) integrin expression in LMH cells. Primers for β-actin and β(1) integrin were previously published (Caprile et al 2009) using GenBank chicken sequences. Conventional PCR amplification was completed using a 25 µl reaction containing: 2.5 µl of cDNA, 0.8 mM dNTPs, 1.6 mM MgCl₂, 2.5 µl 10X Amplitaq Gold Buffer® II, 0.8 U Taq DNA polymerase (Applied Biosystems), and 0.4 µM forward and reverse primers. Each reaction was overlaid with 30 µl Chill Out® wax (Bio-Rad) to prevent evaporation and placed into an MJ Research 60 place thermal cycler (Bio-Rad). The PCR reaction mixture was incubated at 95°C for 10 minutes and 35 cycles of 95°C for 30 seconds, 57°C for 60 seconds, and 72°C for 20 seconds. PCR product was analyzed by agarose gel electrophoresis using the FlashGel® DNA System (Lonza Group Ltd). Amplified products were visualized by UV light transillumination and 100 base pair (bp) molecular weight ladder (Lonza Group Ltd) was concordantly run on the gels to aid in the calculation of the size of the amplified DNA fragments. The expected band size for β-actin and β(1) integrin was 282 and 308 bp, respectively.

Construction and generation of tkRNAi shRNA vectors - The shRNA expression vector, pmbv43, has been previously described (Buttaro and Fruehauf 2010). Pmbv43 contains an expression cassette driven by T7 promoter with cloning sites containing BamHI and Sall restriction enzyme sequences. Upon receiving parent_pmbv43 plasmid from Cambridge Biolabs (Cambridge, MA), the plasmid was digested 2 hours at 37°C in a 50 µl reaction containing: 2 µg pmbv43, 2.0 µl BamHI (New England Biolabs (NEB)), 2.0 µl Sall (NEB), 5.0 µl Buffer 3 (NEB), 0.5 µl 100X bovine serum albumin (NEB), and 24.2 µl molecular water. Digested parent_pmbv43 was subsequently treated with Alkaline Phosphatase, Calf intestinal (NEB) and phenol/chloroform and ethanol precipitated. The resulting 8.4 kb linear parent_pmbv43 plasmid was gel extracted from a 1% agarose gel following gel electrophoresis and isolated using dialysis.

The RNAi targeting sequence of NP was 5' - GGA TCT TAT TTC TTC GGA G - 3' (SEQ ID. NO. 1) and PA was 5' - GCA ATT GAG GAG TGC CTG A - 3' (SEQ ID NO. 2). The complementary/antisense sequence for NP was 5' - CTC CGA AGA AAT AAG ATC C - 3' (SEQ ID NO. 3) and the complementary/antisense sequence for PA was 5' - TCA GGC ACT CCT CAA TTG C - 3' (SEQ ID NO. 4). The DNA template encoding the shRNA specific for NP and PA was: BamHI site - sense sequence-hairpin loop (5' - TTC AAG AGA - 3') - antisense sequence - TTTTTTTTTT - Sall site. In other words, the DNA template encoding the shRNA specific for NP was 5' - GGA TCT TAT TTC TTC GGA GTT CAA GAG ACT CCG AAG AAA TAA GAT CC - 3' (SEQ ID NO. 5) without the poly-T tail and 5' - GGA TCT TAT TTC TTC GGA GTT CAA GAG ACT CCG AAG AAA TAA GAT CCT TTT TTT TTT - 3' (SEQ ID NO. 6) with the poly-T tail. And the DNA template encoding the shRNA specific for PA was 5' - GCA ATT GAG GAG TGC CTG ATT CAA GAG ATC AGG CAC TCC TCA ATT GC - 3' (SEQ ID NO. 7) without the poly-T tail and 5' - GCA ATT GAG GAG TGC CTG ATT CAA GAG ATC AGG CAC TCC TCA ATT GCT TTT TTT TTT - 3' (SEQ ID NO. 8) with the poly-T tail.

Integrated DNA Technologies synthesized the top and bottom strands for the PA DNA oligonucleotide sequence. Each PA oligonucleotide was resuspended to 300 µM and 120 µM of each strand were annealed together in a 20µl reaction. After annealing, the duplex was phosphorylated and ligated into linear pmbv43 for 24 hours at 4°C in a 10 µl reaction containing: 1 µl T4 DNA ligase buffer (NEB), 1 µl of annealed and phosphorylated PA oligonucleotide, 2 µl pmbv43, 1 µl T4 DNA ligase (NEB), and 5 µl molecular water. The resulting ligation mixture was transformed into DH5α™ competent cells (Life Technologies) and the resulting transformed cells were plated onto Luria Broth (LB) plates containing 10 µg/ml kanamycin (Kan) and incubated at 37°C overnight. Resulting colonies were screened by PCR using PA_shRNA specific primers that amplified a 209 bp product according to the following thermal profile: 94°C for 4 minutes, 30 cycles of 94°C for 30 seconds, 45°C for 30 seconds, 72°C for 30 seconds, and a final elongation at 72°C for 10 minutes. A single PCR positive PA_pmbv43 plasmid clone was purified using the PureLink® HiPure Plasmid Maxiprep Kit (Life Technologies) and sequenced to verify proper PA_shRNA insertion. Using standard cloning and plasmid purification methods, the NP_pmbv43 plasmid was commercially synthesized, generated using DH10-beta cells, and purified by the commercial company DNA2.0, Incorporated. Upon receiving NP_pmbv43, the plasmid was resuspended in molecular water to a final concentration of 100 ng/µl. NP_pmbv43, PA_pmbv43, and parent_pmbv43 were transformed into CEQ221 competent cells (NP_pmbv43/CEQ, PA_pmbv43/CEQ, and parent_pmbv43/CEQ vectors) and plated onto Brain Heart Infusion agar containing 25 µg/ml Kan and 50 µg/ml 2,3-Diaminopropionic Acid (BHI/Kan/Dap). Plates were incubated overnight at 37°C and resulting colonies were screened by PCR using sets of NP, PA, and parent_shRNA specific primers. A single PCR positive clone representing NP_pmbv43/CEQ, PA_pmbv43/CEQ, and parent_pmbv43/CEQ was sequenced. Following sequence validation, each clone was subsequently propagated in BHI/Kan/Dap broth. Stocks were generated at mid-log (OD₆₀₀ = 0.4-0.6) and at stationary phase (OD₆₀₀ = 1.0), and frozen back at -80°C in 20% glycerol. A single frozen aliquot from each vector stock representing OD₆₀₀ = 1.0 was thawed for plate enumeration. Briefly, each 1 ml aliquot was thawed, centrifuged for 5 minutes at 5,000 x g, and resuspended in 1 ml of phosphate buffered saline (PBS) containing 100 µg/ml Dap (PBS/Dap). The resulting vectors were diluted in PBS/Dap 1:10, 1:100, 1:1,000, 1:10,000, 1:60,000, 1:90,000, 1:135,000, 1:270,000. A total of 50 µl from each theoretical dilution representing 1:60,000 - 1:270,000 were plated in duplicate on BHI/Kan/Dap agar and incubated overnight at 37°C. Colony counts at each dilution were averaged and used to calculate overall colony forming units per ml (CFU/ml). These enumeration values represented an appropriate viable CFU/ml concentration for NP_pmbv43/CEQ (anti-AIV_NP), PA_pmbv43/CEQ (anti-AIV_PA), and parent_pmbv43/CEQ (anti-AIV_scramble) vector stocks. This system allowed aliquots to be thawed and used directly in all future *in vitro* invasion assays.

In addition to the constructs for NP and PA, constructs were created for the following PB1 and PB2 sequences: PB1 (5' - GAT CTG TTC CAC CAT TGA A - 3') (SEQ ID NO. 11) and PB2 (5' - CGG GAC TCT AGC ATA CTT A - 3') (SEQ ID NO. 12). The complementary/antisense sequence for PB1 was 5' - TTC AAT GGT GGA ACA GAT C - 3' (SEQ ID NO. 13) and the complementary/antisense sequence for PA was 5' - TCA GGC ACT CCT CAA TTG C - 3' (SEQ ID NO. 14). The DNA template encoding the shRNA specific for PB1 and PB2 was: BamHI site - sense sequence-hairpin loop (5' - TTC AAG AGA - 3') - antisense sequence - TTTTTTTTTT - Sall site. In other words, the DNA template encoding the shRNA specific for PB1 was 5' - GAT CTG TTC CAC CAT TGA ATT CAA GAG ATT CAA TGG TGG AAC AGA TC - 3' (SEQ ID NO. 15) without the poly-T tail and 5' - GAT CTG TTC CAC CAT TGA ATT CAA GAG ATT CAA TGG TGG AAC AGA TCT TTT TTT TTT - 3' (SEQ ID NO. 16) with the poly-T tail. And the DNA template encoding the shRNA specific for PB2 was 5' - CGG GAC TCT AGC ATA CTT ATT CAA GAG ATA AGT ATG CTA GAG TCC CG - 3' (SEQ ID NO. 17) without the poly-T tail and 5' - CGG GAC TCT AGC ATA CTT ATT CAA GAG ATA AGT ATG CTA GAG TCC CGT TTT TTT TTT - 3' (SEQ ID NO. 18) with the poly-T tail. It is further contemplated that the order of the sense and antisense sequences within the construct could be reversed such that the order would be BamHI site - antisense sequence-hairpin loop (5' - TTC AAG AGA - 3') - sense sequence - TTTTTTTTTT - Sall site, although it is preferred to place the sense sequence first. In addition, it is contemplated that additional hairpin loop sequences could be used, varying in length from 3 to 23 nucleotides, including hairpin loops having 3, 4, 5, 6, 7, 9, or 23 nucleotides. Thus, the sense and antisense sequences could be separated by a stretch of hairpin loop nucleotides having a length of 3, 4, 5, 6, 7, 9, or 23 nucleotides (in addition to any number of nucleotides between 3 and 23 nucleotides). It is further contemplated that length of the shRNA/siRNA can vary, with a variation in length up to about 30 nucleotides for the resultant siRNA depending upon the composition of the target mRNA sequence. Lastly, it is contemplated that a vector could be constructed to produce more than one siRNA, thereby targeting more than one AIV mRNA, or multiple sequences within a given AIV mRNAm using a single vector. However, producing multiple siRNAs from a single plasmid can result in unequal transcription of the siRNAs.

Intracellular uptake of tkRNAi vectors - To verify bacterial uptake and intracellular invasion into LMH cells, anti-AIV_scramble vector was tagged with a red fluorescent protein (RFP). Briefly, anti-AIV_scramble vector was co-transformed with the RFP prokaryotic expression vector, pE2-Crimson (Clontech), using standard transformation methods. This RFP was chosen because it is a far-red fluorescent protein with fast maturation, high photostability, reduced cytotoxicity, and is expressed from the *lac* promoter to allow for IPTG activation of RFP expression (Bevis and Glick 2002). After growing the anti-AIV_scramble_RFP vectors (RFP-vector), stocks were generated and enumerated, as previously described. LMH cells were seeded in 8-well chamber slides one day prior to invasion to allow cell monolayers to reach 60% confluency. On the day of vector invasion, a 1 ml aliquot of RFP-vector was thawed, centrifuged at 5,000 x g for 5 minutes, and resuspended in PBS/Dap. The RFP vector was then placed on ice and serially diluted 1:4 in Waymouth's MB 752/1 medium containing 2 mM L-Glutamine and 50 µg/ml of Dap starting with 5e7 CFU/ml and ending at 7.8e5 CFU/ml. To prepare LMH cells for invasion and remove antibiotics from the growth medium, medium was aspirated and each well was washed twice with fresh Waymouth's MB 752/1 medium containing 2 mM L-Glutamine and 50 µg/ml of Dap (invasion medium). Rinse medium was aspirated and 1 ml of RFP-vector at each dilution was added to an appropriate well and allowed to incubate for 2 hours at 37°C in the presence of 7% CO₂. One untreated well (Waymouth's MB 752/1 complete medium), one mock-invasion control well (invasion medium only) and one non-RFP invasion control well (anti-AIV_scramble vector at 3.1e6 CFU/ml) was included with each chamber slide experiment. After 2 hours incubation, invasion medium was aspirated and wells were washed twice with Waymouth's MB 752/1 complete medium, before fresh Waymouth's MB 752/1 complete medium was replaced and cells were allowed to continue incubating for an additional 2-24 hours. To fix cells, growth medium was aspirated and wells were rinsed twice with calcium/magnesium free PBS. The chamber was removed from the slide and 10 µl of ProLong® Gold Antifade Reagent with DAPI (Life Technolgies) was added to each well grid and the slide was mounted with a cover slip. The resulting slide was incubated at room temperature in the absence of UV light for 24 hours before images were captured using the Eclipse Ti inverted fluorescent microscope (Nikon Instruments Inc.) at 40X magnification. Filter cubes for DAPI and CY5 were used to visualize the DAPI and RFP fluorophores using 461 and 670 emission, respectively.

Optimal tkRNAi vector concentration - To determine the optimal vector concentration allowable, without concomitant induction of CPE, LMH cells were seeded in 24-well plates one day prior to invasion to allow cell monolayers to reach 80% confluency. Anti-AIV_scramble vector was prepared, as previously described, and diluted in invasion medium to 5e7, 1.25e7, 3.1e6 and 7.8e5 CFU/ml. LMH cells were prepared for invasion, as described above. Rinse medium was aspirated and 1 ml of anti-AIV_scramble vector at each dilution was added to an appropriate well, in triplicate, and allowed to incubate for 2 hours at 37°C in the presence of 7% CO₂. Each plate included an untreated well (Waymouth's MB 752/1 complete medium) and a mock-invasion well (invasion medium only). After 2 hours incubation, invasion medium was aspirated and wells were washed twice with Waymouth's MB 752/1 complete medium before fresh Waymouth's MB 752/1 complete medium was replaced and cells were allowed to continue incubating for an additional 24-48 hours. At multiple time points post invasion, wells were observed for visual signs of CPE, compared to the untreated control wells. The maximum anti-AIV_scramble vector concentration allowable, without inducing CPE, was selected for all subsequent anti-AIV_NP and anti-AIV_PA invasion assays in LMH cells.

Chicken cell invasion with tkRNAi shRNA vectors - LMH cells were seeded in 24-well plates one day prior to invasion to allow cell monolayers to reach 80% confluency. On the day of vector invasion, a 1 ml aliquot of anti-AIV_NP, anti-AIV_PA, and anti-AIV_scramble vector was thawed, centrifuged at 5,000 x g for 5 minutes, and resuspended in PBS/Dap. Each vector was then placed on ice and diluted appropriately for the invasion assay. LMH cells were prepared for invasion, as previously described. Wells were tested in triplicate and included anti-AIV_NP, anti-AIV_PA, anti-AIV_scramble, and a cocktail of anti-AIV_NP + anti-AIV_PA vector (anti-AIV_cocktail). Thus, "cocktail" was a 1:1 mixture of the *E*. *coli* carrying the anti-AIV_NP vector and the *E. coli* carrying the anti-AIV_PA vector. Control wells were also included in triplicate (mock-invasion and untreated). After 2 hours incubation, LMH cells were washed (previously described) and fresh Waymouth's MB 752/1 complete medium was replaced.

Virus infection - Twenty four hours post invasion the LMH cells were prepared for infection by removing the growth medium and washing each well with IM containing 0.25 µg/ml TPCK, as previously described. The wash was removed and a total of 250 µl of IM containing either H8N4 or H6N2 virus at an MOI of 0.01 or 0.001 was added to each appropriate well. Plates were incubated on a rocking platform at 37°C in the presence of 7% CO₂ for 1 hour before removing the virus and replacing with 750 µl/well of fresh IM. Cell culture supernatants were harvested 48 hpi, centrifuged, and frozen at -80°C. Each experiment included a positive control (AIV infected), negative control (untreated), and mock-invasion control (mock-invasion + AIV infected) each tested in triplicate wells.

Evaluation of infectious virus titer - LMH cell culture supernatants were quantitated by end-point titration on MDCK cells as previously described using TCID₅₀/ml calculations. Briefly, the harvested supernatants were thawed, diluted ten-fold, and 100 µl/well of virus suspension was overlaid in triplicate into 96 well plates seeded with MDCKs. Cells were stained with crystal violet 48 hpi and wells with CPE were scored as positive for virus growth. TCID₅₀/mL was calculated by the Reed and Muench mathematical technique (Reed and Muench 1938).

Statistical Analysis - All experiments were repeated a minimum of three times, on different days. Using a statistical significance level of 0.05, the Wilcoxon rank-sum test was performed to compare median viral titers between vector treated and untreated positive control (PC) samples performed on the same day. To determine if adjusted mean LogTCID₅₀/mL values between vector treated and PC samples were statistically different (p < 0.05), data was analyzed using multivariable linear regression, controlling for day. Statistical analyses were performed using the statistical computing software Stata 10 (Statcorp LP).

### RESULTS

Verifying intracellular uptake of tkRNAi vectors in chicken cells - Uptake of the tkRNAi vectors by epithelial cells works by receptor mediated endocytosis when the bacterial surface protein, invasin, interacts with the host receptor, β(1) integrin. It was therefore important to verify the presence and stable expression of β(1) integrin in chicken LMH cells during normal growth conditions and during infection with AIV. Following RNA extraction and cDNA synthesis, the expression of β(1) integrin in LMH cells grown using normal growth conditions, as well as in cells infected with H8N4 virus (MOI 0.01) at 6 and 24 hours post infection (FIG. 2) was observed. These results indicated the tkRNAi vector should appropriately attach to LMH cells, even when the cells are showing signs of CPE and diseased post infection. Tagging the anti-AIV_scramble vector with an RFP verified intracellular expression inside LMH cells at 2 and 24 hours post-invasion (FIG. 3). The maximum RFP-vector concentration allowable, without inducing CPE, was 7.8e5 CFU/ml. Therefore, all subsequent experiments adopted this vector concentration. Together, these results indicate the tkRNAi delivery platform is appropriate for chicken epithelial cells.

Antiviral activity of tkRNAi shRNA vectors in chicken cells - It was previously shown that the NP and PA siRNA sequences align against the H8N4 and H6N2 NP and PA sequences with 100% homology and are devoid of any off-target matching to any known chicken sequence. Prior to infection with H6N2 or H8N4 virus, LMH cells were first treated with vector and following infection culture supernatants were collected for TCID₅₀ analysis.

As expected, the antiviral activity of each vector varies by day but also by virus and MOI used for infection (FIG. 4). However, significant antiviral activity is observed in anti-AIV_NP, anti-AIV_PA, and anti-AIV_cocktail samples from both H6N2 and H8N4 infections. On at least one experimental test day, anti-AIV_NP, anti-AIV_PA, and the anti-AIV_cocktail significantly reduced median virus titers in both viruses at an MOI 0.01, compared to the corresponding PC samples. The vectors appeared to have a slightly less profound antiviral effect at a viral MOI 0.001. Despite a lack of significant differences likely associated with a small sample size, 100%, 89%, and 100% of all NP, PA, and anti-AIV_cocktail vector treatments resulted in lower median viral titers compared to their appropriate untreated PCs, respectively. It should be noted that the anti-AIV_scramble vector also resulted in lower median viral titers, some of which were significantly different from the PC samples (FIG. 4).

By controlling for day, multivariable linear regression (MLR) analysis was used to compare vector treated LMH cells to untreated PC cells across all experimental days. Significant differences in adjusted mean viral titers between vector-treated and untreated PC samples are shown in Table 2. MLR analysis indicates significant differences between all vector treatments (excluding anti-AIV_scramble) and their corresponding PC samples after infection with both H6N2 and H8N4 at MOI 0.01. With the exception of infection with H8N4 MOI 0.001, in all experimental sets the cocktail vector resulted in significantly lower viral titers compared to the appropriate PC titers (p<0.05). The log10 reduction in infectious virus between vector treated and untreated samples were calculated. All vector treated samples resulted in at least 0.8 log10 reduction (6-fold reduction). Most notable was from H8N4 infection at MOI 0.01 after NP (3.8 log or 6,918-fold reduction), PA (2.5 log or 331-fold reduction), and anti-AIV_cocktail vector treatment (4 log or 10,965 fold reduction) as well as H8N4 at MOI 0.001 after anti-AIV_PA vector treatment (3.0 log or 1,000-fold reduction). Interestingly, the scramble vector also showed antiviral activity in all experimental sets, except for infection with H6N2 at MOI 0.001 (Table 2).

**Table 2 - Anti-AIV vector protection in chicken LMH cells as measured by log and fold-reductions in shedding titers.**

| Sample | n | Adjusted Mean^{a} | 95% Cl | Adjusted Mean (untreated) | 95% Cl (untreated) | P-value^{b} | Log reduction^{c} | Fold reduction^{d} |
|---|---|---|---|---|---|---|---|---|
| H8N4 LPAI virus MOI 0.01 | | | | | | | | |
| Anti-AIV_NP | 9 | 1.1 | (0.0,2.6) | 4.9 | (3.7, 6.2) | < 0.001 | 3.8 | 6918 |
| Anti-AIV_PA | 9 | 3.0 | (2.1,3.8) | 5.5 | (4.6, 6.4) | < 0.001 | 2.5 | 331 |
| Anti-AIV_cocktail | 9 | 0.9 | (0.0,2.1) | 4.9 | (3.9, 6.0) | < 0.001 | 4.0 | 10,965 |
| Anti-AIV_scramble | 10 | 2.8 | (2.0,3.5) | 4.9 | (4.1, 5.8) | < 0.001 | 2.1 | 138 |

| H8N4 LPAI virus MOI 0.001 | | | | | | | | |
|---|---|---|---|---|---|---|---|---|
| Anti-AIV_NP | 9 | 8.1 | (6.3, 9.8) | 8.9 | (7.9,10.0) | 0.309 | 0.8 | 6 |
| Anti-AIV_PA | 9 | 5.8 | (4.9, 6.8) | 8.8 | (8.0,9.5) | < 0.001 | 3.0 | 1,000 |
| Anti-AIV_cocktail | 9 | 7.9 | (6.3, 9.5) | 8.9 | (7.9,9.9) | 0.213 | 1.0 | 10 |
| Anti-AIV_scramble | 10 | 7.3 | (6.0, 8.6) | 8.5 | (7.5,9.5) | 0.064 | 1.2 | 16 |

| H6N2 LPAI virus MOI 0.01 | | | | | | | | |
|---|---|---|---|---|---|---|---|---|
| Anti-AIV_NP | 9 | 4.7 | (3.1, 6.2) | 6.3 | (4.8,8.0) | 0.043 | 1.6 | 43 |
| Anti-AIV_PA | 9 | 4.9 | (3.6, 6.2) | 6.3 | (4.8,7.9) | 0.031 | 1.4 | 27 |
| Anti-AIV_cocktail | 9 | 4.4 | (3.0, 5.9) | 6.3 | (4.9,7.8) | 0.013 | 1.9 | 85 |
| Anti-AIV_scramble | 9 | 5.5 | (4.6, 6.3) | 6.3 | (5.3,7.4) | 0.053 | 0.8 | 7 |

| H6N2 LPAI virus MOI 0.001 | | | | | | | | |
|---|---|---|---|---|---|---|---|---|
| Anti-AIV_NP | 9 | 9.2 | (7.5, 9.3) | 10.1 | (9.1, 11.1) | 0.270 | 0.9 | 8 |
| Anti-AIV_PA | 9 | 9.5 | (8.1, 10.9) | 10.5 | (9.4,11.7) | 0.149 | 1.0 | 10 |
| Anti-AIV_cocktail | 9 | 8.4 | (7.1, 9.8) | 10.1 | (9.2,10.9) | 0.023 | 1.7 | 50 |
| Anti-AIV_scramble | 9 | 11.3 | (9.8, 12.8) | 10.5 | (9.3,11.6) | 0.269 | -0.8 | 0 |
| Results are from multivariable linear regression, controlling for day. ^{a} Expressed as adjusted mean LogTCID₅₀/mL. ^{b} Comparing adjusted mean LogTCID₅₀/mL from treated to untreated PC samples (p<0.05). ^{c} Log reduction in mean infectious titer compared to untreated control. ^{d} Fold reduction in geometric mean compared to untreated control. | | | | | | | | |

The objective of this example and the application of its teachings is to provide a means not necessarily to prevent infection, but to reduce the amount of infectious virus shed into the culture supernatants after treating LMH cells with the anti-AIV vectors. In vitro treatment with these antiviral vectors significantly reduced infectious titers following infection with two different AIV subtypes isolated in poultry; H8N4 and H6N2. In all but one sample set (H8N4/MOI 0.001), the cocktail vector significantly reduced viral titers. However, even when a significant difference between vector treated and untreated PC samples was not observed, a notable log10 reduction in infectious titer was observed from all vector treated sample sets. The most potent inhibition of infectious virus shedding occurred after LMH cells were treated with anti-AIV_cocktail and infected with H8N4 or H6N2, resulting in significant log10 reductions ranging from 1.7 - 4.0 (50 - 10,965 fold reduction).

As was noted above, treatment with the scramble vector also reduced infectious titers in all but the H6N2/MOI 0.001 infected sample set. There is a possible explanation for this observation. The innate immune response is part of the host's early defense mechanism. Toll-like receptors (TLRs) play a key role in the innate response by recognizing and binding bacterial components, which are markers of infection to host cells. These bacterial components, also referred to as pathogen-associated molecular patterns (PAMPs), include endotoxins like lipopolysaccharide which act as immune enhancers (Bessler et al 1990). These anti-AIV vectors are delivered by non-pathogenic E. coli bacteria, therefore it is not surprising that these chicken epithelial cells would detect the extracellular presence of these bacterial endotoxins, likely via TLR-4 recognition (Schoen et al 2004). This binding event would lead to the expression of the inflammatory transcription factor, nuclear factor kappaβ, and ultimately the release of downstream proinflammatory cytokines and chemokines as part of the innate response. In this case, these bacterial components could be acting much like a vaccine adjuvant, thereby stimulating the innate response prior to AIV infection and essentially mounting an additional level of protection against viral infection.

This example demonstrates *in vitro* proof of concept and shows the antiviral potential of these novel anti-AIV vectors in an avian tissue model. However, does this data suggest an anti-AIV vector cocktail targeting NP and PA could significantly reduce AIV shedding in chickens? Could this approach translate into an antiviral technology that limits AIV outbreaks and transmission in poultry? Even more, could this represent a transformative approach for controlling influenza in other species? This work represents the first steps towards answering these questions. A goal of this research is to develop the disclosed prophylactic into a novel complement to the traditional AIV vaccine for poultry. As such, the findings from this work have been translated into efforts aimed at assessing the efficacy of these antiviral vectors in experimentally challenged chickens as described in Example 3, immediately below. The long-term goal is to develop an innovative anti-influenza prophylactic that would contribute to the development of a more effective and robust control method for AIV in poultry.

### Example 3 - Prophylactic Treatment of Chicken Populations with Anti-AIV Vectors

In the face of an AIV outbreak, several factors are critical to effectively controlling the spread of virus between and among poultry. These include the speed at which a control method or vaccine is applied, how rapidly a prophylactic works to protect, and the ability to protect against any subtype or strain of AIV. Developing a powerful anti-influenza technology for poultry is a critical step to effectively manage and control the spread of this disease worldwide. Previous work demonstrated the value of using novel anti-AIV vectors targeting the viral NP and PA genes to reduce viral shedding titers *in vitro* but have yet to be tested *in vivo* using experimentally challenged chickens. Vector uptake into chicken respiratory tissues was first assessed using a vectors tagged with fluorescent red protein for visualization. Once vector uptake and a lack of vector associated pathogenicity was demonstrated in chickens, groups of commercial chickens were treated with the cocktail vectors (n=10), a scramble vector (n=10), or a placebo (n=10). Twenty-four hours later, all chickens were challenged with H6N2 virus. Chickens treated prophylactically with the anti-AIV cocktail vectors shed significantly less virus compared to untreated chickens (p<0.05). Likewise, the proportion of chickens shedding virus was significantly less in the anti-AIV cocktail treated chickens compared to the untreated chickens (p<0.05). The anti-AIV cocktails also prevented AIV transmission to sentinel chickens. This work demonstrates *in vivo* proof of concept for using this novel RNAi antiviral technology to protect chickens against AIV replication, viral shedding, and transmission.

### MATERIALS AND METHODS

Animals - Four week-old commercial leghorn chickens were kindly donated by a commercial production farm in Colorado and housed in a biosafety level 2 facility. Upon arrival, birds were randomly allocated to a treatment group by blindly selecting colored coded leg bands placed in a box. On the day of arrival, blood was collected from the brachial vein and an oropharyngeal (OP) swab was taken to verify AIV free status using serology testing and real time RT-PCR. Groups of birds were housed in a room containing separate 12 square-foot suites, in accordance with the Guide for the Care and Use of Laboratory Animals. Negative airflow was maintained to prevent cross contamination between suites, whereby the air was positive to the central corridor of the room during the entire course of the study. Groups of chickens were allowed to roam freely within each suite and feed and water were provided with *ad libitum* access. Room temperature was maintained at 70°F and lights were controlled daily to allow adequate time for daytime activity and resting. Animal care for each experiment was conducted under the approval of the IACUC committee at Colorado State University. To give chickens time to acclimate to their new surroundings, all of the experimental studies commenced at least 4 days after arrival.

Virus - LPAI A/Chicken/Texas/473-2/10 (H6N2) was kindly provided by the United States National Veterinary Services Laboratory (NVSL) in Aimes, IA. The H6N2 virus was chosen due to its history of being isolated from chicken respiratory swabs collected during an outbreak in 2010 occurring in Texas. The virus was propagated and titrated in the allantoic cavity of 10-day old embryonated specific pathogen-free (SPF) chicken eggs for 2 days at 37°C. Briefly, after growing the virus, the amnioallantoic fluid (AAF) was harvested and pooled to represent a single virus stock. To titrate the virus, an aliquot of the stock was diluted ten-fold from 1:10 to 1:10¹⁰ in brain heart infusion broth (BHI) containing 1X antibiotic cocktail (2000 U/ml penicillin G, 4 mg/ml streptomycin, 16 µg/ml gentamycin, 100 U/ml Nyastatin, 650 µg/ml kanamycin) and 0.1 ml was used to inoculate 3 SPF eggs at each dilution. AAF was harvested and the 50% egg infectious (EID₅₀/ml) titer calculated according to the Reed and Muench mathematical formula.

Vector administration - Vector stocks were generated and frozen back at appropriate CFU/ml concentrations (as previously described) so that additional growth and enumerations were not necessary. This essentially allowed vector aliquots to be used directly as the treatment dose appropriate for direct administration to a bird. Briefly, an appropriate volume of vector stock was thawed, centrifuged at 5,000 x g for 5 minutes, and resuspended in PBS containing 50 µg/ml 2,3-Diaminopropionic Acid (Dap). The cocktail vector represented an equal concentration (CFU/ml) in and equal volume (based on total dose volume) of the NP (NP_pmbv43/CEQ) and PA (PA_pmbv43/CEQ) vectors. The vectors were transported on ice to the facility housing the chickens. Vector was administered by the intranasal route (0.3 - 0.5 ml per bird). Each chicken was gently restrained during dosing and an equal volume was administered into each nostril. The bird was restrained for one additional minute to prohibit sneezing and allow the vector to settle into the nasal tissues for efficient invasion.

Virus challenge - To emulate the natural route of infection for AIV in an experimental setting, chickens were inoculated at 5 weeks (± 4 days) of age with 10⁶ EID₅₀/0.1 ml solution of H6N2 virus. Prior to inoculation, virus was diluted in sterile PBS to the desired dose and stored cold until ready for challenge. During challenge, each animal was gently restrained while trained personnel inoculated 50 µl of virus into each nostril. After challenge, each bird was restrained for an additional 1 minute to prohibit sneezing and allow the virus to settle into the nasal tissues for efficient infection. Chickens were evaluated daily for clinical illness

Data and sample collection - Post vector and/or viral inoculation, chickens were inspected daily using the clinical illness (0-4) scoring system: 1 = Mild lethargy evidence by lack of interest in feed, slow movement, and low reaction to external environment; 2 = Mild respiratory disease (open mouth breathing, snicking, sneezing), accompanied by mild lethargy; 3 = Moderate respiratory disease (snicking, sneezing, mild cough, raspy breathing), accompanied by moderate lethargy; and 4 = Severe respiratory disease (coughing, sneezing, labored breathing), accompanied by lack of feeding with limited movement or reaction to the external environment. After challenge with H6N2 virus, OP swabs were collected from infected birds at 2, 3, 4, 5, 6, 7, and 10 days post infection (dpi). Swabs were immediately submerged and rinsed in a falcon tube containing 1 ml brain-heart infusion (BHI) broth storage medium and placed on ice. Personnel collecting the swabs changed gloves and booties between groups to avoid potential cross contamination. Samples were stored at -70°C until subsequent reverse transcriptase quantitative real time PCR (RT-qPCR). At appropriate times, birds were euthanized by CO₂ and tissues collected at necropsy to assess RFP-vector uptake, vector associated pathogenesis, or viral infection.

Detection of viral shedding by RT-qPCR - All OP swab samples collected over the infection period were processed for RT-qPCR to determine H6N2 viral titers. Briefly, total RNA was extracted from 50 µl of OP swab sample using Trizol LS (Life Technologies, Grand Island, NY) and the MagMAX 96 AI/ND Viral RNA isolation kit (Life Technologies) with the KingFisher magnetic particle processor (Thermo Scientific, Waltham, MA). RT-qPCR was performed using the ABI 7500 platform (Applied Biosystems) and with primers and probe specific for conserved sequences in avian influenza matrix gene as previously described (Spackman et al 2002) and in accordance with the NVSL protocol AVSOP1521.01. Primer and probe sequences were as follows: Forward primer (5'-AGA TGA GTC TTC TAA CCG AGG TCG-3'), Reverse primer (5'-TGC AAA AAC ATC TTC AAG TCT CTG-3'), and 5' reporter dye (5'- 6-carboxyfluorescein [FAM]) (5'- FAM-TCA GGC CCC CTC AAA GCC GA-TAMRA-3') 3' reporter dye (6-carboxytetramethylrhodamine [TAMRA]) labeled probe. The RT step conditions were 30 min at 50°C and 15 min at 94°C, followed by 45 cycles of 94°C for 0 s and 60°C for 20 s. The standard curve for virus quantification was generated in triplicate using a series of 10-fold dilutions from 1:10 to 1:10¹⁰ of the H6N2 stock virus from which EID₅₀ equivalents per ml (EID₅₀ eq/ml) sample medium of each RNA sample could be calculated. The limit of detection was determined to be 10¹ EID₅₀/ml (1 log₁₀ EID₅₀/ml) per reaction.

Serology - Upon arrival at the housing facility, serum collected from all birds was tested by the agar gel immune-diffusion (AGID) test and shown to be negative for antibodies to any influenza type A viral antigen. Infected chickens were bled on day 10 post-infection and sera were tested again using the AGID test.

Statistical analysis - Descriptive statistics included median viral titers with 95% confidence intervals (CI), titer peaks and ranges, proportions of chickens shedding, and fold-reductions in shedding titers. Differences in the proportion of chickens shedding virus were analyzed using Fisher's exact test (p<0.05). Differences in the median viral titers between treated and untreated chickens were analyzed using the Wilcoxon rank-sum test (p<0.05). Multivariable linear regression (MLR), controlling for day, was used to determine if adjusted median Log EID₅₀ eq/ml values from OP samples collected from vector treated and untreated PC chickens were statistically different (p < 0.05). Logistic regression analysis was used to determine the odds ratio (OR) of shedding in treated chickens compared to untreated PC chickens (p<0.05), by individual day as well as across all days. Statistical analyses were performed using the statistical computing software Stata 10 (Statcorp LP).

Experimental Design - This work included three initial pilot studies, each requiring the comparative examination of birds, in order to verify anti-AIV vector uptake into chicken respiratory tissues, determine proper anti-AIV vector dose, and verify H6N2 virus is appropriate for challenging the chickens. The findings from these pilot exploratory experiments were an essential part of planning the fourth part of the described *in vivo* work, the final proof of concept (POC) study. The findings from the initial pilot studies were not used to extrapolate to chicken populations due to the limited sample size and lack of power.
***Pilot 1:*** Two groups of two chickens were housed in separate suites and were administered the RFP-vector (parent_pmbv43/CEQ_RFP, previously described) at 1.6e8 CFU in a single 500 µl dose. Each group of two chickens were euthanized by CO₂ inhalation at 15 and 27 hours post RFP-vector treatment. Respiratory tissues were collected at necropsy, including sinus, trachea, pharynx and lungs, and the proventriculus of each animal was similarly collected. Within three hours, these tissues were prepared for fluorescent microscopy and subsequent observation of RFP expression indicating vector adherence and intracellular uptake. Briefly, the tissues were cryogenically frozen and sectioned using a cryostat machine. Tissue sections were mounted on glass coverslips using ProLong® Gold Antifade Reagent with DAPI and covered with a coverslip. After 24 hours incubation at room temperature, slides were maintained at 4°C until images were captured using the Eclipse Ti inverted fluorescent microscope using 461 and 670 emission. The goal of this pilot study was to verify that these *E. coli* bacteria are appropriate vehicles to specifically target and deliver the anti-AIV vectors to the chicken respiratory tissues. Statistical calculation and power justification was not applicable to determine the number of chickens necessary to achieve the goal associated with pilot 1. These were qualitative observations, not aimed at detecting a difference in the measured effects between the two groups but rather interested in determining if these vectors are optimal delivery vehicles to chicken respiratory tissues. A sample size of two chickens per group was adequate to provide multiple tissue samples for the proposed analyses.
***Pilot 2:*** Two groups of five chickens were housed in separate suites and were administered the scramble vector (parent_pmbv43/CEQ, previously described) with one of two doses; 1.36e7 or 3.6e8 CFU per 500 µl dose. Pilot 2 required one group of five chickens to serve as the untreated control group. These chickens were administered 500 µl of PBS/Dap and served as the baseline for comparison to clinical illness scores and histology. Chickens were monitored daily for clinical illness and vector treated chickens euthanized at day 3 (n=2), day 7 (n=2), and at day 14 (n=1). The untreated control birds were euthanized at day 3 (n=1), day 7 (n=2), and at day 14 (n=2). During necropsy, chickens were assessed for gross signs of pathogenesis and/or inflammation indicative of bacterial infection and sinus, trachea, pharynx, lungs, and the proventriculus tissues were collected. Tissue were fixed in 10% neutral buffered formalin solution, sectioned, and stained with hematoxylin and eosin. The goal of this pilot study was to verify these vectors are well-tolerated at the two administered doses, without a concomitant induction of gross or microscopic signs of epithelial damage at day 3, 7 and 14 post treatment, compared to the untreated tissues. Statistical calculation and power justification was not applicable to determine the number of chickens necessary to achieve the goal associated with pilot 2. These were qualitative observations, aimed at determining the highest vector dose tolerable, based on lowest average clinical illness and least observable gross or microscopic signs of epithelial damage. A sample size of five chickens per group was adequate to calculate the average clinical illness scores in each group. Pilot 2 was necessary to determine the optimal anti-AIV vector dose for the final POC experiment.
***Pilot 3:*** One group of ten chickens was challenged with LPAI H6N2 virus. Chickens were evaluated daily for clinical illness and OP swabs collected to detect viral shedding using RT-qPCR. At 10 dpi chickens were euthanized. The purpose of pilot 3 was to verify chicken susceptibility to infection following H6N2 challenge by way of clinical illness scores and/or detection with RT-qPCR.

***Proof Of Concept (POC):*** Three groups of ten chickens, housed in three separate suites, were administered 3.6e8 CFU of vector or a placebo in a 300 µl dose. These groups included group 1 = anti-AIV cocktail vector, group 2 = scramble vector, and group 3 = positive control (PBS/Dap solution). All birds were challenged via intranasal (IN) route with H6N2 virus at 20 hours post treatment (± 2 hours). Chickens were evaluated daily for clinical illness and OP swabs were collected. At 10 dpi chickens were euthanized and necropsied for signs of disease and collection of sinus, trachea, pharynx, lungs, liver, and spleen. One-half of the tissue section was fixed in 10% neutral buffered formalin solution, while the remaining half of fresh tissue was stored at -80°C. This work required three groups of animals (n=10 for each group) to statistically determine differences in viral shedding between chickens treated with the anti-AIV vectors and untreated positive control chickens. Estimated sample sizes were calculated to achieve at least 80% power with a level of significance of 95% using the statistical analyses previously indicated (Wilcoxon rank-sum test, Fisher's exact test, multivariable linear regression, and logistic regression). The most critical measurement is the EID₅₀/ml, as this value represents a viral titer, which indicates viral shedding. Therefore, power calculation was based on expected EID₅₀/ml measurements. It was estimated that the mean viral titer in the untreated chickens would be 5.5 log EID₅₀/ml with standard deviation (SD) ± 0.5 log EID₅₀/ml (Swayne and Beck, 2005). For this work, at least 5-fold reduction (0.7 log EID₅₀/ml difference) in viral titers would be deemed statistically significant. Therefore, the estimated mean viral titer for the treated chickens would be 4.8 log EID₅₀/ml with SD ± 0.5 log EID₅₀/ml. The calculated effect size was 1.4 ((largest mean - smallest mean)/SD = (5.5-4.8)/0.5). Using the Stata code, fpower, the output table indicated a sample size between 9 and 10 was required to achieve a power of 0.8 with α = 0.05. A sample size of ten was sufficient to demonstrate a ≥ 5-fold reduction in viral shedding associated with chickens treated with the cocktail vector expressing viral specific shRNAs compared to untreated PC chickens or scramble vector treated chickens.

***Sentinels:*** Two additional 4-week old commercial chickens were added to the shipment of thirty animals for the final POC study. In lieu of euthanizing these extra animals, they were housed among the ten H6N2 challenged chickens that had additionally received the anti-AIV cocktail vector. These sentinels were in direct contact with these infected animals and shared the same food and water. The goal of using these two sentinel chickens was to monitor possible transmission.

### RESULTS

RFP-vector localization in chicken respiratory tissues - In pilot 1, animals were euthanized and tissues were collected 15 and 27 hours after chickens were treated with the RFP-vector. These tissues were prepared for fluorescent microscopy and images of treated and untreated tissues were captured. This RFP is generated by the prokaryotic expression vector, pE2-Crimson (Clontech). This fluorescent protein is very bright, photostable, and noncytotoxic due to its solubility in the cytosol of host cells (Strack et al 2011). It was also chosen because of its far-red excitation and emission properties, allowing differentiation between this red fluorescent signal and possible background due to auto-fluorescence. Shown in FIG. 5, the RFP expressing bacteria appear to localize to the epithelium of the sinus tissues, trachea, and lungs at both time points. This is in contrast to a lack of red expression visible in those tissues collected from PBS/Dap treated chickens.

Optimal vector dose and associated pathogenicity - In pilot 2, chickens were administered one of two doses of scramble-vector and were observed for 14 days. Between days 1-14 post-treatment, birds in all groups did not show signs of any distress or clinical illness. At both vector doses, groups of chickens were euthanized at day 3 (n=2), 7 (n=2) and 14 (n=1) post-treatment and at necropsy all tissues appeared normal compared to tissues from birds treated with the placebo (PBS/Dap). No obvious pathogenesis was observed in the proventriculus tissues. Histological assessment indicated vector treatment was not associated with respiratory tissue pathogenesis when compared to PBS/Dap treated tissues. Histology did indicate minor underlying disease present in some treated and untreated tissues, which may have been present in these commercial chickens prior to the start of the study. Therefore, it was difficult to determine if disease was associated with the vector, the treatment volume (500 µl), or if the animals came from a population previously diseased.

For this reason, it was decided to repeat this work using birds given only the higher vector dose (3.6e8 CFU per 500 µl dose) compared to birds treated with 500 µl PBS/Dap, birds that were untreated, and birds arriving fresh from the commercial operation. The last group of birds was included to determine if disease found in the untreated birds was present prior to arrival or if disease developed after arrival to the housing facility. Briefly, two birds were randomly allocated to each of the four groups. With the exception of the fresh arrival birds, which were euthanized and necropsied on arrival, birds in the remaining three groups were treated with vector, PBS/Dap, or nothing and observed for 14 days. On day 14, all birds were euthanized and necropsied. At necropsy, none of the birds in any of the groups displayed any signs of disease or abnormalities and the pathologist analyzing the histological slides was blinded to remove any potential bias. Histology revealed very little pathology or macrophage infiltration in the lungs, trachea, and air sacs of any bird that would indicate an adverse reaction to the vector or dose volume, especially in the in the airways and interstitium. There was mild perivascular inflammation noted in some of the tissues, but the presentation was minimal and random among all groups. Furthermore this presentation is not unusual given these birds are commercial and not SPF.

Susceptibility to H6N2 challenge - In pilot 3, ten chickens were challenged with H6N2 virus. OP swabs were collected and clinical illness noted. While mortality was not anticipated, one chicken was found dead at 7 dpi. At 8 dpi, 6/9 remaining chickens were AIV seropositive after AGID testing and chickens were euthanized at 10 dpi. The purpose of this pilot was to verify H6N2 infection and shedding after experimental challenge. Therefore, OP swabs collected on 3, 4 and 5 dpi were the first and only group of samples tested using RT-qPCR. Results revealed shedding in 7/10, 10/10, and 8/10 chickens at 3, 4, and 5 dpi, respectively. This data was sufficient to verify chicken susceptibility to H6N2 challenge and no further RT-qPCR testing was conducted on remaining samples.

Vector antiviral activity in experimentally challenged chickens - In the final POC study, groups of birds were prophylactically treated with the cocktail vector, scramble vector, or given a placebo (PBS/Dap) prior to infection with H6N2 virus. OP swabs were collected and clinical illness was noted daily. At 8 dpi, one chicken in the PC group died. This was the only mortality in all groups. The remaining chickens were euthanized at 10 dpi. Blood collected prior to euthanasia revealed 4/10, 6/10, and 6/9, cocktail, scramble and PC chickens were AIV seropositive, respectively. H6N2 challenge produced subclinical illness, as none of the chickens presented any notable signs of disease. As such, the clinical illness scoring system was not used and scores were not analyzed.

Vector protection as measured by a cumulative reduction in median virus titer and the proportion of positive OP swabs out of the total swabs collected from vector treated compared to PC chickens is shown in Table 3. This crude data table represents differences across the entire study, without adjusting for day. A significant difference in median titer between the cocktail treated (1.5, 95% Cl = 0, 3.0) and the PC chickens (4.1, 95% Cl = 2.8, 4.8) (Wilcoxon rank sum, p<0.0001) is shown. Likewise, there is a significant difference in the median titer between scramble treated (3.2, 95% Cl = 0, 3.9) and PC chickens (p=0.003). There is a significant difference in the proportion of positive OP swabs collected from the cocktail group, 36/70 (51%), compared with 58/70 (83%) from the PC group (Fisher's exact test, p<0.0001). This significant difference was also observed between scramble treated (42/70, 60%) and PC birds (p=0.002). Although there is no significant difference in the cumulative median titer or proportion of positive swabs between the cocktail and scramble groups, in both measurements, the antiviral effect in the cocktail group is more profound. Table 3 indicates vector protection, but does not reveal which day during infection these significant differences are occurring.

| Table 3 - Vector protection as measured by reduction in virus titer and number of positive OP swabs from vector treated chickens. | | |
|---|---|---|
| Group | (95% Cl)^{a} | Positive OP Swabs^{b} |
| Cocktail | 1.5 (0, 3.0) | 36/70 (51%) |
| Scramble | 3.2 (0, 3.9) | 42/70 (60%) |
| PC | 4.1 (2.8. 4.8) | 58/70 (83%) |

| | P-value¹ | P-value² |
|---|---|---|
| Cocktail PC | < 0.0001 | < 0.0001 |
| Scramble PC | 0.003 | 0.002 |
| Cocktail Scramble | 0.247 | 0.198 |
| ^{a} Represents log EID₅₀ eq/mL from all OP swabs collected over infection period. ^{b} Number of positive/total OP swabs tested by RT-qPCR. ¹ Wilcoxon rank-sum test for difference in median titer values (p<0.05). ² Fisher's exact test for difference in proportion of positive swabs (p < 0.05). | | |

Table 4 portrays vector protection on each day. For each treatment group, titer peak and range, median titer, and shedding proportions on each day-post infection are shown. The Wilcoxon rank-sum test for a difference in median titer indicates significant differences between cocktail treated and PC chickens at days 3 and 4 post-infection. No significant difference in median titers on any individual day are observed between the scramble treated and PC chickens. There is a significant difference in the proportion of birds shedding in the cocktail group (5/10) compared to the PC group (10/10) at 3 dpi (p=0.016). However, the differences in shedding proportions are insignificant between the scramble treated and PC group on any individual day.

| Table 4 - Vector protection as measured by reduction in virus titer and number of vector treated chickens shedding H6N2 by day. | | | | | | | | |
|---|---|---|---|---|---|---|---|---|
| Days Post-infection with H6N2 Virus | | | | | | | | |
| Group | 0 | 2 | 3 | 4 | 5 | 6 | 7 | 10 |
| COCKTAIL (n=10) | | | | | | | | |
| Titer Peak^{a} | 0 | 4.86 | 4.75 | 4.79 | 5.32 | 5.60 | 5.96 | 4.95 |
| Titer Range^{b} | 0 | 2.2 - 4.9 | 2.3 - 4.8 | 2.4 - 4.8 | 1.5 - 5.3 | 1.4-5.6 | 3.2 - 6.0 | 2.3 - 4.9 |
| Median Titer | 0 | 2.19 | 1.15 | 1.20 | 0.77 | 2.19 | 1.60 | 0 |
| (95% CI) | | (0, 3.31) | (0, 4.43) | (0, 4.61) | (0, 4.82) | (0,4.85) | (0, 5.27) | (0, 4.39) |
| P-value^{†} | N/A | 0.105 | 0.05 | 0.042 | 0.089 | 0.147 | 0.18 | 1.0 |
| Shedding proportion | 0/10 | 6/10 | 5/10 | 5/10 | 5/10 | 6/10 | 5/10 | 4/10 |
| P-value^{††} | N/A | 0.500 | 0.016 | 0.070 | 0.175 | 0.152 | 0.070 | 0.328 |

| SCRAMBLE (n=10) | | | | | | | | |
|---|---|---|---|---|---|---|---|---|
| Titer Peak^{a} | 0 | 5.03 | 4.96 | 6.00 | 5.17 | 5.14 | 4.88 | 3.75 |
| Titer Range^{b} | 0 | 2.6 - 5.0 | 2.5- 5.0 | 3.7 - 6.0 | 3.1 - 5.2 | 4.3 - 5.1 | 1.4 - 4.9 | 1.5-3.8 |
| Median Titer | 0 | 2.97 | 3.22 | 3.92 | 3.52 | 4.39 | 3.83 | 0 |
| (95% CI) | | (0, 4.43) | (0, 4.77) | (0, 5.23) | (0,4.78) | (0,4.84) | (0, 4.66) | (0,3.28) |
| P-value^{†} | N/A | 0.279 | 0.289 | 0.322 | 0.145 | 0.223 | 0.102 | 0.658 |
| Shedding proportion | 0/10 | 6/10 | 7/10 | 6/10 | 6/10 | 6/10 | 7/10 | 4/10 |
| P-value^{††} | N/A | 0.500 | 0.105 | 0.152 | 0.314 | 0.152 | 0.291 | 0.328 |

| + CONTROL (n=10) | | | | | | | | |
|---|---|---|---|---|---|---|---|---|
| Titer Peak^{a} | 0 | 5.79 | 6.42 | 5.81 | 5.81 | 6.02 | 5.85 | 3.97 |
| Titer Range^{b} | 0 | 3.0 - 5.8 | 1.5 - 6.4 | 1.7 - 5.8 | 2.8 - 5.8 | 2.1 - 6.0 | 1.8 - 5.9 | 1.4 - 4.0 |
| Median Titer | 0 | 3.90 | 4.05 | 4.77 | 4.57 | 4.65 | 4.82 | 1.38 |
| (95% CI) | | (0, 5.25) | (2.1,5.2) | (2.0,5.3) | (0.9, 5.5) | (2.3, 5.4) | (2.0, 5.6) | (0, 2.9) |
| Shedding proportion | 0/10 | 7/10 | 10/10 | 9/10 | 8/10 | 9/10 | 9/10 | 5/9* |
| ^{a} Titers represent log EID50 eq/mL values. ^{b}Represents only birds shedding virus. ^{†}Wilcoxon rank-sum test for difference in median titer compared to positive control group(p<0.05). ^{††}One-sided Fisher's exact test for difference in proportion of chickens shedding in vector treated compared to positive control group (p<0.05). * One bird died between 7 and 10 dpi. | | | | | | | | |

Median H6N2 virus titers and the proportion of chickens shedding virus at each daily interval are shown in FIG. 6. Looking at the plot of median titer by day representing all chickens in each group (including those not shedding), there a distinct trend in the load of virus shed between the cocktail chickens and those in the scramble and PC group (FIG. 6A). Between day 2 and 7 post-infection, median titers in both the scramble and PC groups appear to rise, while the median titer in the cocktail group drops and continues to decline until day 5 post-infection. The titer in the cocktail group does briefly rise at 6 and 7 dpi, but the load of virus shed is still much lower than the virus load shed from the scramble and PC chickens. A less profound trend is observed when the median titer by day representing those chickens shedding in each group is plotted (FIG. 6B). Here each median titer represents the daily proportion of chickens shedding from Figure 6C. With the exception of day 3 and 10 post-infection, the daily median load of virus shed is less in the cocktail compared to the PC shedding birds. Despite this apparent switch in observed virus titer between the cocktail and PC group on these two days, it is important to recognize that still a greater number of birds are shedding (FIG. 6C) on day 3 and 10 in the PC group (10/10 and 5/9) compared to the cocktail group (5/10 and 4/10).

As is expected and observed, median shedding titers from birds in each group varies by day. Significant differences between crude median titers are observed (Table 3), however to determine significant differences across all days post-infection, it is necessary to control for day in the analysis. Vector protection as measured by a reduction in median titer is analyzed using MLR, controlling for day (Table 5). Regardless of the day post infection, there is a significant difference in median titer in the PC chickens compared to the cocktail (p<0.0001) and compared to the scramble (p=0.002) chickens. In the majority of analyses and comparisons made up to this point, the cocktail treatment protection tended to be superior compared to the scramble treatment. However using MLR and controlling for day, this difference is insignificant (p=0.251).

| Table 5 - Vector protection as measured by reduction in median virus titer across all days. | |
|---|---|
| | P-value^{†} |
| Cocktail vs. PC | < 0.0001 |
| Scramble vs. PC | 0.002 |
| Cocktail vs. Scramble | 0.251 |
| ^{†} Multivariable linear regression for difference in median titer, controllingfor day (p<0.05). | |

Simple and multiple logistic regression analyses were used to estimate vector protection as measured by the odds of shedding H6N2 virus in the positive control chickens compared to vector treated chickens (Table 6). ORs, 95% CIs, and level of significance (p<0.05) were computed for each individual day and after controlling for day (across all days). With the exception of 3 dpi, the odds of shedding on an individual day in the PC chickens is not significant compared to the cocktail or scramble treated chickens. The odds of shedding could not be calculated on day 3 post-infection because 10/10 (100%) PC chickens were shedding virus. When controlling for day, the odds of shedding H6N2 among PC chickens is 4.83 (95% Cl = 2.17, 10.72) greater compared to cocktail treated chickens (p<0.0001). Compared to the scramble vector treated chickens, the odds of shedding detectable virus across all days in the PC chickens is slightly less (3.48; 95% Cl = 1.57, 7.86), but still significant (p=0.003). Albeit insignificant, across all days the odds of shedding virus in the scramble group is 1.42 greater compared to the odds of shedding in the cocktail group (p=0.303).

| Table 6 - Vector protection as measured by the odds of shedding virus in untreated positive control chickens. | | | | | | | | | |
|---|---|---|---|---|---|---|---|---|---|
| | | Untreated Positive Control | | | | Scramble Vector | | | |
| Reference Group | Dpi | OR^{a} | LCL^{b} | UCL^{c} | P-value^{d} | OR | LCL | UCL | P-value |
| Cocktail Vector | Across all days^{e} | 4.83 | 2.17 | 10.72 | < 0.0001 | 1.42 | 0.73 | 2.8 | 0.303 |
| | 2 | 1.55 | 0.24 | 9.91 | 0.640 | 1.0 | 0.17 | 5.98 | 1.0 |
| | 3 | --* | -- | -- | -- | 2.33 | 0.37 | 14.61 | 0.365 |
| | 4 | 9.0 | 0.81 | 100.14 | 0.074 | 1.5 | 0.25 | 8.82 | 0.654 |
| | 5 | 4.0 | 0.55 | 29.1 | 0.171 | 1.5 | 0.25 | 8.82 | 0.654 |
| | 6 | 6.0 | 0.53 | 67.65 | 0.147 | 1.0 | 0.17 | 5.98 | 1.0 |
| | 7 | 9.0 | 0.81 | 100.14 | 0.074 | 2.33 | 0.37 | 14.61 | 0.365 |
| | 10 | 2.25 | 0.38 | 13.47 | 0.374 | 1.0 | 0.17 | 5.98 | 1.0 |
| Scramble Vector | Across all days^{e} | 3.48 | 1.54 | 7.86 | 0.003 | | | | |
| | 2 | 1.55 | 0.24 | 9.91 | 0.640 | | | | |
| | 3 | -- | -- | -- | -- | | | | |
| | 4 | 6.0 | 0.53 | 67.65 | 0.147 | | | | |
| | 5 | 2.67 | 0.36 | 19.71 | 0.337 | | | | |
| | 6 | 6.0 | 0.53 | 67.65 | 0.147 | | | | |
| | 7 | 3.86 | 0.33 | 45.57 | 0.284 | | | | |
| | 10 | 2.25 | 0.38 | 13.47 | 0.374 | | | | |
| ^{a} Odds ratio calculated using logistic regression analysis.^{b} Lower 95% confidence level.^{c} Upper 95% confidence level.^{d} Calculated using simple and multiple logistic regression analysis (p<0.05).^{e} Controlling across all days.* 10/10 chickens shedding in untreated PC group; OR is not applicable. | | | | | | | | | |

Vector protection as measured by log reduction and fold reduction in replication and shedding titers is shown in Table 7. Differences in median titer (log EID₅₀ eq/mL) between vector-treated and PC chickens was calculated as the log reduction. Using the geometric median values, fold reductions in virus titers were calculated. Both of these reduction measurements were computed across all days (using the unadjusted median titer) and for each individual day. Across all days, the cocktail treatment is associated with a 2.6 median log reduction (398-fold reduction) in virus titer shed compared to the PC chickens. At each individual day post-infection, the cocktail treatment resulted in a measureable median log reduction. Most notable is at days 3, 4, 5, 6, and 7 post-infection, in which viral shedding is between 2.5 and 3.8 median logs lower (288 to 6309-fold lower) compared to the PC chickens. Log reductions in viral shedding are much less pronounced in the scramble treated chickens. However, overall still resulted in a 0.9 median log reduction (7.9-fold reduction) and at each individual day at least a 0.3 median log reduction (1.8-fold reduction). Most notable for the scramble vector treatment was at day 10 post infection (1.4 median log reduction), which represents the least potent reduction observed in the cocktail group, also at day 10 post infection.

| Table 7 - Vector protection as measured by log and fold-reduction in shedding titers. | | | |
|---|---|---|---|
| Treatment Group | Dpi | Log Reduction ^{a} | Fold Reduction ^{b} |
| Cocktail | Across all days ^{c} | 2.6 | 398.1 |
| | 2 | 1.7 | 51.3 |
| | 3 | 2.9 | 794.3 |
| | 4 | 3.6 | 3,715.4 |
| | 5 | 3.8 | 6,309.6 |
| | 6 | 2.5 | 288.4 |
| | 7 | 3.2 | 1,659.6 |
| | 10 | 1.4 | 24.0 |
| Scramble | Across all days | 0.9 | 7.9 |
| | 2 | 0.9 | 8.5 |
| | 3 | 0.8 | 6.8 |
| | 4 | 0.9 | 7.1 |
| | 5 | 1.1 | 11.2 |
| | 6 | 0.3 | 1.8 |
| | 7 | 1.0 | 9.8 |
| | 10 | 1.4 | 24.0 |
| ^{a} Reduction in median log EID₅₀ eq/mL titer compared to positive control.^{b} Reduction in geometric median compared to positive control. ^{c} Based on crude median log EID₅₀ eq/mL not adjusted for day. | | | |

Sentinels - Naïve chickens (n=2) were housed with the cocktail treated birds from the time of vector administration to 10 days post H6N2 challenge. From both of these birds, only one OP positive swab was detected at day 7 post introduction (1.5 log EID50 eq/mL). All other swabs collected between day 2 and 10 post infection from these sentinel birds were negative. Overall, 13/14 (92.8%) of OP swabs collected from these contact birds were negative.

This study is the first to show efficient delivery and bacteria-mediated invasion into avian sinus, trachea, and lung tissues using the tkRNAi delivery platform. RFP-vector uptake was assessed at two time points, 15 and 27 hours post treatment. After delivery and invasion to the chicken respiratory tissues, it is unknown how long these vectors would actively supplying shRNA to these recipient non-phagocytic epithelial cells. To show initial efficacy, it was decided to assess and verify transient vector uptake to simulate viral protection within 24 hours. Images captured in FIG. 5 showing RFP-vector localization to the respiratory tissues at both time points was sufficient to conclude that vector delivery and theoretically, the unloading of shRNA would precede viral challenge.

Intranasal administration of the scramble vector was not associated with any observed pathogenesis within 14 days post treatment as compared to placebo (PBS/Dap) and untreated birds. Lack of any observed clinical illness and favorable histopathology results suggests these vectors were well-tolerated at the dose administered. As a follow-up additional studies could be performed. Pilot 2 did not test if tissue specific pathogenesis would be present if sequential vector doses were administered across several days. Also not evaluated in this work are alternative ways of quantifiably measuring vector treatment response, including a drop in egg production for layer birds and lack of weight gain in broilers. In future work, this prophylactic technology could include assessments beyond 14 days and after multiple doses. However, these vectors are Dap auxotrophic and once administered, unable to survive or proliferate outside of a supplemented medium. It is doubtful a chronic inflammatory response would incur beyond 14 days due to a lack of any gross or microscopic tissue damage at 2 weeks post treatment. These initial pilot results are promising and indicate this tkRNAi system is appropriate for avian respiratory tissue delivery and does not trigger an inflammatory response that the avian host is unable to overcome within 14 days.

Pilot 3 verified that H6N2 virus at the challenge dose was infectious in 5 week -Id commercial layer chickens. This dose (10⁶ EID₅₀/0.1 mL) is common for LPAI vaccine efficacy trials and other experimental challenge studies in chickens (Claes et al 2013, Pantin-Jackwood et al 2012, Abbas et al 2011). Together pilot 1, 2, and 3 verified vector uptake within 24 hours, identified an appropriate vector dose, and an optimal LPAI challenge virus. These parameters were incorporated into the final POC study.

Three groups of ten chickens where prophylactically administered the cocktail, scramble or placebo vector and challenged with H6N2 20 hours (±2) post treatment. The crude data presented in Table 3 indicates significant differences in shedding titers and the proportion of positive swabs collected across all days post-infection when comparing the cocktail or scramble treated birds to the PC birds. These results are further supported when MLR analysis is conducted, controlling for day. It is interesting to expand these results by day to identify where and allude to why these differences are occurring between treatment groups. Following H6N2 challenge, it appears that the proportion of chickens shedding on 2 dpi is relatively similar; meaning the success of experimental infection was relatively equal in all groups. Yet, of birds shedding on day 2, shedding titers are lower in the cocktail compared to both the scramble and PC birds. Additionally, individual bird shedding (not presented) indicates one cocktail treated bird was shedding at 2 dpi, but stop shedding at a detectable level on subsequent days. This intuitively makes sense because the vector's mode of action is aimed at preventing replication, not infection.

At day three, while shedding proportions in the scramble (7/10) and PC (10/10) groups appear to spike, the cocktail group significantly drops to 5/10 and plateaus through days 3, 4, and 5. In support of this observation, shedding titers are significantly different at days 3 and 4 post infection between cocktail treated and PC chickens. Again, looking at shedding trends (FIG. 6A), at day 3 through 5 post-infection median titers drop in the cocktail group, but continue to increase out to day 7 post infection in both the scramble and PC groups. These observations might explain the NP and PA shRNA antiviral activity, interfering with efficient viral replication during the infectious period. There is an alternative way to interpret these observations. In the cocktail treated group, more chickens were shedding virus at 2 days post infection compared to days 3-5. This might indicate vector protection would be more effective if administered > 24 hours prior to challenge, allowing greater time for shRNA unloading and processing.

Shedding titers and proportions by day support a proposed model of vector protection. Following viral entry and infection, the vector interferes with viral replication, inhibiting the proliferation of infectious virions that go on to infect neighboring cells and tissues. This reduces the infectious viral load within the respiratory tissues, thereby decreasing viral shedding in each chicken. Because AIV is primarily transmitted through direct contact in chickens, this prevents subsequent infection of susceptible birds in that group. Together these findings imply cocktail vector treatment disrupts efficient viral replication and shedding.

Shedding duration directly influences the infectious period or the time from when virus is first detected to when the virus is no longer detected. It is provides another way to assess vector protection. However, it can be argued that shedding duration plays a lesser role in transmission compared to shedding titer. An animal might be shedding over a longer duration, but shedding titers too low to support successful transmission. Additionally, bird density might not support transmission, despite longer shedding periods. For that reason, shedding duration is not typically used as a measurement for evaluating vaccine efficacy. This study was terminated at 10 dpi, despite continued shedding among chickens in each group. Therefore, had the study continued beyond 10 days it would be possible to precisely measure the vector's effect on shedding duration. The cocktail vectors reduced oropharyngeal viral shedding titers, but do not appear to limit the duration of shedding. Perhaps this indicates the vectors appropriately provided transient protection early on in the infection cycle, but also might indicate a need to administer a subsequent vector dose, much like a booster vaccine. This might further protect susceptible birds from those infected, thereby blocking the transmission chain.

In addition to assessing shedding duration and differences in shedding titers and shedding proportions, the odds of shedding virus in the PC chickens compared to vector treated chickens provides an alternative way to measure vector protection. Controlling across all days post infection, the PC chickens were 4.83 times more likely to shed H6N2 virus, compared to the cocktail treated chickens (p<0.0001). PC chickens were also more likely to shed virus compared to the scramble treated chickens. While these odds ratios provide greater support for vector protection, they are not a standardized method of evaluating vaccine efficacy in the industry.

The quantitative reduction in virus replication in the respiratory tract is a critical measurement used to evaluate a prophylactic's capacity to limit virus transmission and control disease (Swayne and Kapczynski 2008). To be deemed clinically relevant, an AIV vaccine must demonstrate a minimum reduction in replication and shedding titers of 10² EID₅₀ (2 log or 100-fold) less virus from respiratory tracts in vaccinated compared to nonvaccinated birds (Suarez et al 2006), and/or the difference should be statistically significant (Swayne et al 1997). While the scramble vector did not demonstrate this minimum required reduction, it did demonstrate a statistically significant difference in shedding titers across all days (Table 5). With the exception of day 2 and 10 post-infection, shedding titers from the cocktail vector demonstrated a minimum of 2.5 log (288-fold) and a maximum of 3.8 log (6,309-fold) less virus compared to the PC birds. Across all days, the anti-AIV cocktail group was shedding 398-fold less infectious virus compared to the PC group. These measurements were all statistically significant. Based on both of these measurable criteria, the cocktail vector treatment was well within the parameters to be regarded as providing clinically relevant protection against AIV.

Several other measurements are used to assess vaccine protection. Vaccinated birds are often tested for quantifiable resistance after challenge with 10² to 10⁵ EID₅₀ greater dose compared with unvaccinated birds (Swayne and Kapczynski 2008). In addition, clinical illness as defined by a drop in egg production in layer birds can quantifiably measure protection. Egg production does not begin until 18-20 weeks of age. To mimic the age at which the industry typically applies vaccine regimens, the birds utilized in this study were 4-6 weeks of age. Therefore measuring egg production would not have been possible. Future studies utilizing this vector system would use both of these assessments to quantifiably measure vector protection. The prevention of mortality is another direct measurement of vaccine protection. LPAI is by definition low pathogenic and not often associated with mortality. However, this H6N2 virus is well-adapted to chickens, contributing to 10% mortality in the PC chickens and chickens challenged in pilot 3. In contrast, the cocktail and scramble vectors both appeared to prevent mortality, as none of the birds receiving either vector died following H6N2 infection.

Finally, prevention of contact transmission is a direct method to demonstrate a vaccine's protective efficacy and propensity to limit field transmission. Therefore, clinically relevant reductions in shedding can be demonstrated by showing reduced spread to contact birds, a practice often requested by national veterinary biologic regulatory agencies. This is difficult to assess in experimental settings because factors that play a role in transmission are not standardized. These factors include bird density, ventilation, humidity, temperature, challenge virus and dose, route of administration, age at challenge, virus-host adaptation, and room sanitation. The intranasal 50% bird infectious dose (BID₅₀) has been suggested as a way to quantifiably assess the potential for AIV to initiate infection and support transmission in chickens challenged in a laboratory setting (Tumpey et al, 2004). In the present study, it can be said that the birds were challenged with an appropriate BID₅₀ to initiate infection and support transmission because greater than 50% of the PC chickens were shedding virus. Two naïve sentinel chickens, housed with the cocktail treated birds, served as a way to monitor viral spread within this group at a density of 1.0 sq-ft per bird. With the exception of one positive swab at 7 days post introduction, no bird-to-bird transmission was detected in the two sentinels. The titer detected from this one swab was just above the limit of detection of the RT-qPCR assay. It is possible that this positive sample was truly due to H6N2 transmission. However, it is also possible this sporadic positive swab was a result of cross contamination from gloves during collection or viral RNA during the extraction procedure.

In one laboratory-based transmission study, researchers experimentally challenged 3 week-old SPF chickens (n=10) with LPAI A/Ck/HN/1/98 (H9N2) (Guan et al 2013). When housed at a density of 0.5 sq-ft per bird, mean OP viral titers of 2.1 log 10 EID₅₀ eq/mL at 2 dpi was sufficient for transmission to naïve chickens (n=2). A second study intranasally challenged 46 week old SPF leghorn chickens (n=10) with 0.1 mL containing 10⁶ EID₅₀ of A/chicken/CA/1255/02 (H6N2) virus and 3 dpi added two sentinel birds to monitor contact transmission (Pantin-Jackwood et al 2012). In the challenged chickens, mean OP titers ± SD (log10 EID₅₀ eq/mL) at 2 and 4 dpi were 4.8 ±0.5 and 5.3 ±0.5, respectively. At 4 days post introduction, both sentinels were positive (3.7 and 3.1 log10 EID₅₀ eq/mL titers). While bird density is not presented in this study, shedding titers in the challenged birds are similar to those titers detected in the current PC chickens at day 2 and 4 post infection. Vector protection as measured by reduced transmission potential would have been better illustrated had sentinel birds also been housed with the PC chickens. Unfortunately, this was not possible as only two extra birds were provided by the commercial farm. In addition to using an appropriate BID₅₀, these previous studies indicate shedding titers would have been adequate to support transmission if sentinels had been housed among PC chickens. Regardless, it appears that the cocktail vector successfully inhibited transmission to these contact birds. To make any valid inferences about capacity of these anti-AIV vectors to break the transmission chain, a standardized laboratory contact-transmission model is required to more accurately reproduce a field or on farm environment.

Mucosal surfaces of the poultry respiratory tract are known gateways of entry for AIV (Zarzaur and Kudsk 2001). Because AIV invades the mucosal surfaces, targeted delivery of these vectors to the mucosal respiratory tracts in chickens is an added benefit to targeting viral replication. In this way, these vectors could provide strong protection at the first line of defense for the host. However, concerns over immune activation are important to consider, as there are pros and cons to activating an immune response in the respiratory tissues. Unlike the natural microflora present in poultry GI tracts, it is unlikely respiratory tissues have developed immune tolerance against bacterial components. A moderate immune response could be beneficial, especially against influenza infection. This might help explain the antiviral activity associated with scramble vector treatment.

However, it is important to avoid stimulating a strong mucosal immune response. This could interfere with the efficacy of this intranasally applied vector, especially if multiple doses are necessary for better protection against AIV. After concluding the POC work, it was learned that these 4 week old pullets had been vaccinated at 7 days of age with the Poulvac® *E*. *coli* modified-live vaccine (Zoetis, Florham Park, New Jersey). This newly approved vaccine has been studied to determine its broad-spectrum protection mechanism against avian pathogenic *Escherichia coli* (APEC). When administered by spray or drinking water, it appears the vaccine stimulates the production of APEC specific immunoglobulin A in the mucosa tissues and proliferation of CD8 memory cells, both of which indicate class I MHC activation leading to a cellular, rather than a humoral response (Filho et al 2013). Activation of a cellular immune response would help prevent future tissue invasion by APEC, especially in the respiratory tract, as this is the primary route of APEC infection (Sadeyen et al 2014). Vaccination may have resulted in mucosal inflammation at 4 weeks of age. Perhaps Poulvac® *E*. *coli* vaccination explains the underlying disease observed in pilot 2 (first round) and resulted in observed inflammation and lymphoid aggregates in the respiratory tissues of both control and vector treated birds.

Besides concerns over Poulvac® *E*. *coli* vaccine induced mucosal inflammation, an obvious caveat to this *E*. *coli* vaccine is if it affected vector efficacy by preventing vector invasion of the respiratory epithelium via priming a cell-mediated response. While this concern is valid, it can be addressed in several ways. The bacteria used in the anti-AIV vector system are the non-mobile *E*. *coli* strain CEQ221. These bacteria are a K-12 derivative, phenotypically rough, and while their lipopolysaccharide has a complete core structure, they lack O antigen (Liu and Reeves 1994). This means these vectors are defective in LPS O-antigen biosynthesis and may lack efficient LPS expression resulting in lower levels of LPS on their outer membranes. It is therefore unlikely CEQ221 would induce a robust immune response. CEQ221 are non-pathogenic, invasive intracellular bacteria and have been genetically engineered to target mucosal epithelial cells. These invasive *E*. *coli* require (β)1Integrin expression on the surface of mucosal epithelial cells. Entry is dependent on this invasin-(β)1Integrin receptor interaction allowing for bacterial uptake via receptor mediated endocytosis (Xiang et al 2006, Isberg and Barnes 2001, Conte et al 1994, Isberg and Leong 1990). If not for the genetically engineered bacterial expression of the invasin protein, these CEQ221 bacteria would otherwise be extracellular and recognized by professional antigen presenting cells (APCs). Epithelial cells are not typically characterized as professional APCs. Because of this specific mucosal epithelium targeting system, dendritic cells and other APCs are less likely to interact or respond to these vectors. CEQ221 is a Dap auxotroph and undergoes rapid lysis after invasion into host cells. Even if CEQ221 were invasive to APCs and other immune cells, the expression of LLO after bacterial lysis in the phagosome would disrupt the fusion between the cell's phagosome and lysosome. This disruption would block class I MHC presentation and prevent activation of a cell-mediated immune response via CD8 memory cell proliferation (Grillot-Courvalin et al 1998). Therefore, these CEQ221 vectors are not well suited to develop a cell-mediated or even a humoral immune response. These characteristics, or lack thereof, might explain this vector's ability to evade the cellular response generated by the Poulvac® *E*. *coli* vaccine in these commercial chickens.

Had the animals utilized in this study been SPF birds, vector protection might have been more directly measured. However, to best mimic the efficacy of this vector system in the field, an argument can be made for using these commercial birds. Ultimately, it is difficult to decipher the effects this Poulvac® *E*. *coli* vaccine may have played on the anti-AIV vectors in this preliminary pilot work. However, if the *E. coli* vaccine had any deleterious effects this would suggest the results from this work underestimate vector protection against AIV in chickens.

While results suggest the cocktail vector provided greater protection, it is not unusual that the scramble vector alone has antiviral capabilities. These bacterial vectors are characteristically LPS rough, however even very low levels of LPS can act as immune enhancers (Bessler et al 1990). LPS is commonly recognized by host tissues and stimulates an innate immune response. LPS recognition can lead to downstream signaling pathways that stimulate type I IFN production. This scenario is probable given some past research related to CEQ508 *E*. *coli* bacteria, a derivative of the CEQ221 strain that serves as anti-AIV vector delivery vehicle. In previous work (Steinbach et al 2010), oral dosing with CEQ508 did not elicit a significant increase in circulating pro-inflammatory cytokines in mice as compared to LPS injections. However, these bacterial vectors did stimulate very low levels of these cytokines, including TNF-α, IL-6, MCP-1, and IL-12. These are all key cytokines involved in the innate immune response, a well-known cellular defense mechanism against viral infection, specifically influenza (Garcia-Sastre and Biron 2006). When these bacterial vectors were administered via the intranasal route to the chickens, it is quite possible that these vectors triggered an innate response, resulting in an added protection against H6N2 and replication and shedding. The production of pro-inflammatory cytokines during this innate activation would increases the ability of any uninfected host cells to resist new infection by newly generated virus.

As has been presented and discussed, there are several ways to measure and describe vector protection from this POC work. Whether looking at differences in shedding proportions, shedding titers, or odds ratios both by day and across all days, a similar pattern has appeared. Compared to the positive controls, chickens treated prophylactically with the anti-AIV cocktail vector were protected following experimental challenge with H6N2. Although analyses were insignificant on any individual day, overall (across all days) significant protection was linked to scramble vector treatment. Albeit, protection from the scramble vector was less profound, suggesting the cocktail vector has greater antiviral potential. This added protection is likely due to the dual antiviral action of NP and PA shRNA.

On average between days 3-7 post infection, 48% of the cocktail vector treated chickens were not shedding. In contrast, only 10% of the chickens in the PC group were not shedding. Results analyzing reductions in shedding titer showed that the cocktail vector provided clinically relevant protection.

As stated previously, the long-term goal of this work is to develop this anti-influenza technology into a prophylactic that would contribute to the development of a more effective and robust control method for AIV in poultry. Successfully developing this technology would not only reduce the economic burden outbreaks have on the industry and developing countries, it would directly reduce the transmission risk to humans and provide proof-of-concept to the scientific and medical community for developing a similar anti-influenza prophylactic for humans. As with developing any new technology, additional studies are needed and should not be underestimated. This work demonstrates the protective efficacy of these anti-AIV vectors against avian influenza in chickens and provides a strong argument for the continued evaluation of this technology.

| Table 8 - AIV Target Sequences | | |
|---|---|---|
| Gene | Sense Strand Sequence (5'-3') | SEQ ID NO. |
| NP | GGA TCT TAT TTC TTC GGA G | 1 |
| PA | GCA ATT GAG GAG TGC CTG A | 2 |
| PB1 | GAT CTG TTC CAC CAT TGA A | 9 |
| PB2 | CGG GAC TCT AGC ATA CTT A | 10 |

| **Table 9 - AIV Sequences** | | | |
|---|---|---|---|
| **AIV siRNA** | **AIV mRNA target** (**5' -3')** | **siRNA sense strand (5' - 3')** | **siRNA antisense strand (5' - 3')** |
| NP-siRNA | GGATCTTATTTCTTC GGAG | GGATCTTATTTCTTCG GAG-dTdT | CTCCGAAGAAATAAGAT CC-dTdT |
| PA-siRNA | GCAATTGAGGAGTG CCTGA | GCAATTGAGGAGTGC CTGA-dTdT | TCAGGCACTCCTCAATTG C-dTdT |
| PB1-siRNA | GATCTGTTCCACCA TTGAA | GATCTGTTCCACCATT GAA-dTdT | TTCAATGGTGGAACAGA TC-dTdT |
| PB2-siRNA | CGGGACTCTAGCAT ACTTA | CGGGACTCTAGCATA CTTA-dTdT | TAAGTATGCTAGAGTCCC G-dTdT |

| **Table 10** - **AIV** shRNA **sequences** | |
|---|---|
| **AIV shRNA** | **5'- sense strand - hairpin loop (TTCAAGAGA) - antisense strand - poly T tail - 3' (top strand)** |
| NP-shRNA (top) | GGATCTTATTTCTTCGGAG-TTCAAGAGA-CTCCGAAGAAATAAGATCC- TTTTTTTTTT |
| NP-shRNA (bottom) | CCTAGAATAAAGAAGCCTC-AAGTTCTCT-GAGGCTTCTTTATTCTAGG- AAAAAAAAAA |
| PA-shRNA (top) | GCAATTGAGGAGTGCCTGA-TTCAAGAGA-TCAGGCACTCCTCAATTGC- TTTTTTTTTT |
| PA-shRNA (bottom) | GCGTTAACTCCTCACGGACT-AAGTTCTCT-AGTCCGTGAGGAGTTAACG- AAAAAAAAAA |
| PB1-shRNA | GATCTGTTCCACCATTGAA-TTCAAGAGA-TTCAATGGTGGAACAGATC- |
| (top) | TTTTTTTTTT |
| PB1-shRNA (bottom) | CTAGACAAGGTGGTAACTT-AAGTTCTCT-AAGTTACCACCTTGTCTAG- AAAAAAAAAA |
| PB2-shRNA (top) | CGGGACTCTAGCATACTTA-TTCAAGAGA-TAAGTATGCTAGAGTCCCG- TTTTTTTTTT |
| PB2-shRNA (bottom) | GCCCTGAGATCGTATGAAT-AAGTTCTCT-ATTCATACGATCTCAGGGC- AAAAAAAAAA |

| **Table 11 - AIV shRNA sequences** | |
|---|---|
| **AIV shRNA** | **5'- sense strand - hairpin loop (TTCAAGAGA) - antisense strand - poly T tail - 3' (top strand)** |
| NP-shRNA (top) | GGATCTTATTTCTTCGGAG-TTCAAGAGA-CTCCGAAGAAATAAGATCC- TTTTTTTTTT |
| NP-shRNA (bottom) | CCTAGAATAAAGAAGCCTC-AAGTTCTCT-GAGGCTTCTTTATTCTAGG- AAAAAAAAAA |
| PA-shRNA (top) | GCAATTGAGGAGTGCCTGA-TTCAAGAGA-TCAGGCACTCCTCAATTGC- TTTTTTTTTT |
| PA-shRNA (bottom) | GCGTTAACTCCTCACGGACT-AAGTTCTCT-AGTCCGTGAGGAGTTAACG- AAAAAAAAAA |
| PB1-shRNA (top) | GATCTGTTCCACCATTGAA-TTCAAGAGA-TTCAATGGTGGAACAGATC- TTTTTTTTTT |
| PB1-shRNA (bottom) | CTAGACAAGGTGGTAACTT-AAGTTCTCT-AAGTTACCACCTTGTCTAG- AAAAAAAAAA |
| PB2-shRNA (top) | CGGGACTCTAGCATACTTA-TTCAAGAGA-TAAGTATGCTAGAGTCCCG- TTTTTTTTTT |
| PB2-shRNA (bottom) | GCCCTGAGATCGTATGAAT-AAGTTCTCT-ATTCATACGATCTCAGGGC- AAAAAAAAAA |
| Scramble-shRNA (top) | AGGTCGCTTAGTCGAGAAG-TTCAAGAGA-CTTCTCGACTAAGCGACCT- TTTTTTTTTT |
| Scramble-shRNA (bottom) | TCCAGCGAATCAGCTCTTC-AAGTTCTCT-GAAGAGCTGATTCGCTGGA- AAAAAAAAAA |

### GLOSSARY OF CLAIM TERMS

The term "administration" and variants thereof (e.g., "administering" a compound) in reference to a compound of the invention means introducing the compound into the system of the subject in need of treatment. When a compound of the invention is provided in combination with one or more other active agents (e.g., an AIV vaccine, etc.), "administration" and its variants are each understood to include concurrent and sequential introduction of the compound and other agents.

As used herein, the term "composition" is intended to encompass a product comprising the specified ingredients in the specified amounts, as well as any product which results, directly or indirectly, from combination of the specified ingredients in the specified amounts.

The term "therapeutically effective amount" as used herein means that amount of active compound or pharmaceutical agent that elicits the biological or medicinal response in a tissue, system, animal or human that is being sought by a researcher, veterinarian, medical doctor or other clinician. In reference to AIV infection, an effective amount comprises an amount sufficient to prevent contracting the disease or to reduce the severity of the disease as evidenced by clinical symptoms, viral titer or virus shedding from the subject, or as evidenced by the ability to prevent or reduce transmission between animals. In some embodiments, an effective amount is an amount sufficient to delay onset of symptoms or to prevent the disease. In some embodiments, an effective amount is an amount sufficient to lower viral titers and/or reduce viral shedding. An effective amount can be administered in one or more doses.

The term "treating AIV" or "treatment of AIV" refers to administration to an animal, specifically poultry, at risk for contracting AIV and refers to an effect that alleviates the disease by inhibiting replication of the AIV, but also to an effect that results in the reduction of clinical symptoms, viral titer and/or viral shedding.

As used herein, "treatment" refers to obtaining beneficial or desired clinical results. Beneficial or desired clinical results include, but are not limited to, any one or more of: alleviation of one or more symptoms, , diminishment of extent of AIV, stabilized (i.e., not worsening) state of AIV, preventing or delaying spread (e.g., shedding) of the AIV, preventing, delaying or slowing of AIV progression, and/or maintain weight/weight gain or the production of eggs, thereby preserving animal productivity. The methods of the invention contemplate any one or more of these aspects of treatment.

A "subject in need of treatment" is an animal at risk for contracting AIV.

A "pharmaceutically acceptable" component is one that is suitable for use with humans and/or animals without undue adverse side effects (such as toxicity, irritation, and allergic response) commensurate with a reasonable benefit/risk ratio.

A "safe and effective amount" refers to the quantity of a component that is sufficient to yield a desired therapeutic response without undue adverse side effects (such as toxicity, irritation, or allergic response) commensurate with a reasonable benefit/risk ratio when used in the manner of this invention.

As used throughout the entire application, the terms "a" and "an" are used in the sense that they mean "at least one", "at least a first", "one or more" or "a plurality" of the referenced components or steps, unless the context clearly dictates otherwise. For example, the term "a cell" includes a plurality of cells, including mixtures thereof.

The term "and/or" whereever used herein includes the meaning of "and", "or" and "all or any other combination of the elements connected by said term".

As used herein, the term "comprising" is intended to mean that the products, compositions and methods include the referenced components or steps, but not excluding others. "Consisting essentially of" when used to define products, compositions and methods, shall mean excluding other components or steps of any essential significance. Thus, a composition consisting essentially of the recited components would not exclude trace contaminants and pharmaceutically acceptable carriers. "Consisting of" shall mean excluding more than trace elements of other components or steps.

As used herein, the term "invasive" when referring to a microorganism, e.g., a bacterium or bacterial therapeutic particle (BTP), refers to a microorganism that is capable of delivering at least one molecule, e.g., an RNA or RNA-encoding DNA molecule, to a target cell. An invasive microorganism can be a microorganism that is capable of traversing a cell membrane, thereby entering the cytoplasm of said cell, and delivering at least some of its content, e.g., RNA or RNA-encoding DNA, into the target cell. The process of delivery of the at least one molecule into the target cell preferably does not significantly modify the invasion apparatus.

Invasive microorganisms include microorganisms that are naturally capable of delivering at least one molecule to a target cell, such as by traversing the cell membrane, e.g., a eukaryotic cell membrane, and entering the cytoplasm, as well as microorganisms which are not naturally invasive and which have been modified, e.g., genetically modified, to be invasive. In another preferred embodiment, a microorganism that is not naturally invasive can be modified to become invasive by linking the bacterium or BTP to an "invasion factor", also termed "entry factor" or "cytoplasm-targeting factor". As used herein, an "invasion factor" is a factor, e.g., a protein or a group of proteins which, when expressed by a non-invasive bacterium or BTP, render the bacterium or BTP invasive. As used herein, an "invasion factor" is encoded by a "cytoplasm-targeting gene". Invasive microorganisms have been generally described in the art, for example, U.S. Pat. Pub. Nos. US 20100189691 A1 and US20100092438 A1 and Xiang, S. et al., Nature Biotechnology 24, 697 - 702 (2006).

In a preferred embodiment the invasive microorganism is E. *coli,* as taught in the Examples of the present application. However, it is contemplated that additional microorganisms could potentially be adapted to perform as tkRNAi vectors for the delivery of anti-AIV siRNA. These non-virulent and invasive bacteria and BTPs would exhibit invasive properties, or would be modified to exhibit invasive properties, and may enter a mammalian host cell through various mechanisms. In contrast to uptake of bacteria or BTPs by professional phagocytes, which normally results in the destruction of the bacterium or BTP within a specialized lysosome, invasive bacteria or BTP strains have the ability to invade non-phagocytic host cells. Naturally occurring examples of such intracellular bacteria are *Yersinia, Rickettsia, Legionella, Brucella, Mycobacterium, Helicobacter, Coxiella, Chlamydia, Neisseria, Burkolderia, Bordetella, Borrelia, Listeria, Shigella, Salmonella, Staphylococcus, Streptococcus, Porphyromonas, Treponema,* and *Vibrio,* but this property can also be transferred to other bacteria or BTPs such as E. *coli, Lactobacillus, Lactococcus,* or Bifidobacteriae, including probiotics through the transfer of invasion-related genes (P. Courvalin, S. Goussard, C. Grillot-Courvalin, C.R. Acad. Sci. Paris 318, 1207 (1995)). Factors to be considered or addressed when evaluating additional bacterial species as candidates for use as tkRNAi vectors include the pathogenicity, or lack thereof, of the candidate, the tropism of the candidate bacteria for the target cell, or, alternatively, the degree to which the bacteria can be engineered to deliver siRNA to the interior of a target cell, and any synergistic value that the candidate bacteria might provide by triggering the host's innate immunity.

Avian influenza viruses are diseases that pose significant threats to animal and human health and are a source of genetic diversity that permits the emergence of pandemic influenza. As used herein, avian influenza is influenza that adversely affects at least some poultry by causing flu symptoms, and possibly death, in the poultry. The fact that an influenza can be carried by some poultry without producing flu symptoms or adversely affecting the health of the poultry does not alter the fact that the influenza is an avian influenza and is a disease as long as the influenza adversely affects the health of at least some poultry. As used herein, a poultry that contracts avian influenza virus and that does not exhibit symptoms and/or that functions as a carrier of the virus is still said to have contracted the avian influenza disease. A poultry contracts avian influenza virus when the virus is in the body of the poultry.

As used herein, a disease is prevented before or after poultry is exposed to the disease, if (1) a medicament composition is administered to an animal internally (by ingestion, inhalation, injection, etc.), topically (on the skin for absorption into the body), or otherwise, and (2) the medicament composition prevents the poultry from contracting the disease and experiencing symptoms normally associated with the disease, or, if the poultry contracts the disease and experiences or doesn't experience in varying degrees of severity some or all of the symptoms normally associated with the disease, the poultry recovers from the disease to a normal healthy state. In a similar manner, the risk of spreading Avian Influenza is reduced if a poultry's shedding of the virus is reduced (e.g. reduced shedding titers) irrespective of whether the poultry survives infection with Avian Influenza virus. As discussed above, one of the principal goals of the present invention is to halt or reduce the spread of Avian Influenza, which reduction can be addressed by reducing transmission from infected poultry to other animals or humans.

As used herein, a disease is treated if a medicament composition is administered to poultry after the poultry has contracted a disease. As noted above, a poultry that contracts a disease may or may not exhibit symptoms associated with the disease.

The symptoms produced in poultry by avian influenza range from a mild illness to a highly contagious and rapidly fatal "highly pathogenic" form of the disease that can produce severe epidemics. Highly pathogenic avian influenza is characterized by sudden onset, severe and rapid death, and a mortality that can approach 100%.

By "kit" is intended any manufacture (e.g., a package or a container) comprising at least one reagent, e.g., a pH buffer of the invention. The kit may be promoted, distributed, or sold as a unit for performing the methods of the present invention. Additionally, the kits may contain a package insert describing the kit and methods for its use. Any or all of the kit reagents may be provided within containers that protect them from the external environment, such as in sealed containers or pouches.

The practice of the present invention may employ, unless otherwise indicated, conventional techniques and descriptions of organic chemistry, polymer technology, molecular biology (including recombinant techniques), cell biology, biochemistry, and immunology, which are within the skill of the art. Such conventional techniques include polymer array synthesis, hybridization, ligation, and detection of hybridization using a label. Specific illustrations of suitable techniques can be had by reference to the examples herein above. However, other equivalent conventional procedures can, of course, also be used. Such conventional techniques and descriptions can be found in standard laboratory manuals such as Genome Analysis: A Laboratory Manual Series (Vols. I-IV*),* Using Antibodies: A Laboratory Manual*,* Cells: A Laboratory Manual*,* PCR Primer: A Laboratory Manual*,* and Molecular Cloning: A Laboratory Manual (all from Cold Spring Harbor Laboratory Press), Stryer, L. (1995) Biochemistry (4th Ed.) Freeman, N.Y., Gait, "Oligonucleotide Synthesis: A Practical Approach" 1984, IRL Press, London, Nelson and Cox (2000), Lehninger, Principles of Biochemistry 3rd Ed., W.H. Freeman Pub., New York, N.Y. and Berg et al. (2002) Biochemistry, 5th Ed., W.H. Freeman Pub., New York, N.Y.,

In an advantageous embodiment, the kit containers may further include a pharmaceutically acceptable carrier. The kit may further include a sterile diluent, which is preferably stored in a separate additional container. In another embodiment, the kit further comprising a package insert comprising printed instructions directing the use of a combined treatment of an pH buffer and the anti-AIV agent as a method for treating Avian Influenza in a subject. The kit may also comprise additional containers comprising additional anti-influenza agents (e.g. amantadine, rimantadine and oseltamivir), agents that enhance the effect of such agents, or other compounds that improve the efficacy or tolerability of the treatment.

## Claims

1. A nonpathogenic *E*. *coli* bacterium comprising a prokaryotic vector, said vector comprising a DNA molecule encoding one or more siRNAs and a promoter to control transcription of the siRNA, wherein the siRNAs interfere with one or more Avian Influenza viral RNA molecules and wherein the bacterium is engineered to express at least one invasion factor.

2. The nonpathogenic *E*. *coli* bacterium according to claim 1 wherein the invasion factor is at least one *inv* gene and at least one *hlyA* gene.

3. The nonpathogenic *E*. *coli* bacterium according to any of claims 1 or 2 wherein the siRNA interferes with an NP, PA, PB1 or PB2 mRNA.

4. The nonpathogenic *E*. *coli* bacterium according to any one of claims 1 or 2 wherein the siRNA comprises a sequence selected from the group consisting of SEQ ID No. 1, SEQ ID No. 2, SEQ ID NO. 3, SEQ ID NO. 4, SEQ ID NO. 11, SEQ ID NO. 12, SEQ ID NO. 13 and SEQ ID NO. 14.

5. The nonpathogenic *E*. *coli* bacterium according to any one of claims 1 or 2 wherein the prokaryotic vector is a plasmid that encodes a transcript comprising a sequence selected from the group consisting of SEQ ID No. 5, SEQ ID No. 7, SEQ ID No. 15, and SEQ ID No. 17.

6. The nonpathogenic *E*. *coli* bacterium according to any one of claims 1 or 2 wherein the promoter is selected from the group consisting of a T7 promoter, a T3 promoter and an SP6 promoter.

7. The nonpathogenic *E*. *coli* bacterium according to any one of claims 1 or 2 wherein the siRNA targets a conserved sequence, wherein the conserved sequence is shared by a plurality of Avian Influenza subtypes.

8. The nonpathogenic *E*. *coli* bacterium according to any one of claims 1 or 2 wherein the prokaryotic vector is a plasmid encoding the *inv* gene and the *hlyA* gene.

9. A pharmaceutical composition comprising the nonpathogenic *E*. *coli* bacterium according to any one of claims 1 to 8.

10. The pharmaceutical composition according to claim 9, wherein the composition is suitable for intranasal administration or spraying as an aerosol suitable for inhalation.

11. The pharmaceutical composition according to any one of claims 9 or 10, comprising a plurality of *E*. *coli* bacterium populations wherein each population of *E*. *coli* is engineered to express a different siRNA molecule that interferes with one or more Avian Influenza viral RNA molecules.

12. A pharmaceutical composition comprising a first nonpathogenic *E*. *coli* bacterium according to any one of claims 1 to 8 and a second nonpathogenic *E. coli* bacterium according to any one of claims 1 to 8, the first nonpathogenic *E*. *coli* bacterium comprising a prokaryotic vector, said vector comprising a DNA molecule encoding a promoter and a first siRNA, wherein the first encoded siRNA interferes with one or more Avian Influenza viral RNA molecules and the promoter controls the transcription of the first siRNA, the second nonpathogenic *E*. *coli* bacterium comprising a prokaryotic vector, said vector comprising a DNA molecule encoding a promoter and a second siRNA, wherein the second encoded siRNA interferes with a different Avian Influenza viral RNA molecule and the promoter controls the transcription of the second siRNA, the first and second bacteria in a pharmaceutically acceptable carrier.

13. The pharmaceutical composition according to claim 12 wherein the first encoded siRNA sequence comprises SEQ ID No. 3 and the second encoded siRNA sequence comprises SEQ ID No. 4.

14. The pharmaceutical composition according to any of claims 9 to13, for use in treating or reducing the risk of spreading Avian Influenza in a bird or poultry.

15. A prokaryotic vector according to claim 1, wherein the prokaryotic vector is a plasmid encoding one or more siRNAs, a promoter to control transcription of the siRNAs, and at least one invasion factor, wherein the siRNAs interfere with the mRNA of an Avian Influenza Virus.

16. The prokaryotic vector according to claim 15 wherein the siRNA interferes with an NP, PA, PB1 or PB2 mRNA.

17. The prokaryotic vector according to claim 15 wherein the siRNA comprises a sequence selected from the group consisting of SEQ ID No. 1, SEQ ID No. 2, SEQ ID NO. 3, SEQ ID NO. 4, SEQ ID NO. 11, SEQ ID NO. 12, SEQ ID NO. 13 and SEQ ID NO. 14.

## Patentansprüche

1. Nicht pathogenes E.-coli-Bakterium, das einen prokaryotischen Vektor umfasst, wobei der Vektor ein DNA-Molekül umfasst, das für eine oder mehrere siRNAs und einen Promotor zum Steuern der Transkription der siRNA codiert, wobei die siRNAs ein oder mehrere virale RNA-Moleküle der Vogelgrippe interferieren, und wobei das Bakterium so manipuliert ist, dass es zumindest einen Invasionsfaktor exprimiert.

2. Nicht pathogenes E.-coli-Bakterium nach Anspruch 1, wobei der Invasionsfaktor zumindest ein inv-Gen und zumindest ein hlyA-Gen ist.

3. Nicht pathogenes E.-coli-Bakterium nach einem der Ansprüche 1 oder 2, wobei die siRNA eine NP-, PA-, PB1-oder PB2-mRNA interferiert.

4. Nicht pathogenes E.-coli-Bakterium nach einem der Ansprüche 1 oder 2, wobei die siRNA eine Sequenz umfasst, die aus der Gruppe ausgewählt ist, bestehend aus SEQ ID No.: 1, SEQ ID No. 2, SEQ ID NO. 3, SEQ ID NO. 4, SEQ ID NO. 11, SEQ ID NO. 12, SEQ ID NO. 13 und SEQ ID NO. 14.

5. Nicht pathogenes E.-coli-Bakterium nach einem der Ansprüche 1 oder 2, wobei der prokaryotische Vektor ein Plasmid ist, das für ein Transkript codiert, umfassend eine Sequenz, die aus der Gruppe ausgewählt ist, bestehend aus SEQ ID No. 5, SEQ ID No. 7, SEQ ID No. 15 und SEQ ID No. 17.

6. Nicht pathogenes E.-coli-Bakterium nach einem der Ansprüche 1 oder 2, wobei der Promotor aus der Gruppe ausgewählt ist, bestehend aus einem T7-Promotor, einem T3-Promotor und einem SP6-Promotor.

7. Nicht pathogenes E.-coli-Bakterium nach einem der Ansprüche 1 oder 2, wobei die siRNA auf eine konservierte Sequenz abzielt, wobei die konservierte Sequenz von einer Mehrzahl von Vogelgrippe-Subtypen geteilt wird.

8. Nicht pathogenes E.-coli-Bakterium nach einem der Ansprüche 1 oder 2, wobei der prokaryotische Vektor ein Plasmid ist, das für das inv-Gen und das hlyA-Gen codiert.

9. Pharmazeutische Zusammensetzung, die das nicht pathogene E.-coli-Bakterium nach einem der Ansprüche 1 bis 8 umfasst.

10. Pharmazeutische Zusammensetzung nach Anspruch 9, wobei die Zusammensetzung für eine intranasale Verabreichung oder Sprühung als für die Inhalation geeignetes Aerosol geeignet ist.

11. Pharmazeutische Zusammensetzung nach einem der Ansprüche 9 oder 10, die eine Mehrzahl von E.-coli-Bakterium-Populationen umfasst, wobei jede Population von E. coli so manipuliert ist, dass sie ein unterschiedliches siRNA-Molekül exprimiert, das ein oder mehrere virale RNA-Moleküle der Vogelgruppe interferiert.

12. Pharmazeutische Zusammensetzung, die ein erstes nicht pathogenes E.-coli-Bakterium nach einem der Ansprüche 1 bis 8 und ein zweites nicht pathogenes E.-coli-Bakterium nach einem der Ansprüche 1 bis 8 umfasst, wobei das erste nicht pathogene E.-coli-Bakterium einen prokaryotischen Vektor umfasst, wobei der Vektor ein DNA-Molekül umfasst, das für einen Promotor und eine erste siRNA codiert, wobei die erste codierte siRNA ein oder mehrere virale RNA-Moleküle der Vogelgruppe interferiert und der Promotor die Transkription der ersten siRNA steuert, wobei das zweite nicht pathogene E.-coli-Bakterium einen prokaryotischen Vektor umfasst, wobei der Vektor ein DNA-Molekül umfasst, das für einen Promotor und eine zweite siRNA codiert, wobei die zweite codierte siRNA ein unterschiedliches virales RNA-Molekül der Vogelgruppe interferiert und der Promotor die Transkription der zweiten siRNA steuert, wobei das erste und das zweite Bakterium in einem pharmazeutisch verträglichen Träger vorliegen.

13. Pharmazeutische Zusammensetzung nach Anspruch 12, wobei die erste codierte siRNA-Sequenz SEQ ID No. 3 umfasst und die zweite codierte siRNA-Sequenz SEQ ID No. 4 umfasst.

14. Pharmazeutische Zusammensetzung nach einem der Ansprüche 9 bis 13 zur Verwendung bei der Behandlung oder Verringerung des Risikos einer Ausbreitung von Vogelgruppe bei einem Vogel oder bei Geflügel.

15. Prokaryotischer Vektor nach Anspruch 1, wobei der prokaryotische Vektor ein Plasmid ist, das für eine oder mehrere siRNAs, einen Promotor zum Steuern der Transkription der siRNAs und zumindest einen Invasionsfaktor codiert, wobei die siRNAs die mRNA eines Vogelgruppevirus interferieren.

16. Prokaryotischer Vektor nach Anspruch 15, wobei die siRNA eine NP-, PA-, PB1- oder PB2-mRNA interferiert.

17. Prokaryotischer Vektor nach Anspruch 15, wobei die siRNA eine Sequenz umfasst, die aus der Gruppe ausgewählt ist, bestehend aus SEQ ID No.: 1, SEQ ID No. 2, SEQ ID NO. 3, SEQ ID NO. 4, SEQ ID NO. 11, SEQ ID NO. 12, SEQ ID NO. 13 und SEQ ID NO. 14.

## Revendications

1. Bactérie *E. coli* non pathogène comprenant un vecteur procaryote, ledit vecteur comprenant une molécule d'ADN codant pour un ou plusieurs ARNsi et un promoteur pour contrôler la transcription de l'ARNsi, dans laquelle les ARNsi interfèrent avec une ou plusieurs molécules d'ARN viral de la grippe aviaire et dans laquelle la bactérie est génétiquement modifiée pour exprimer au moins un facteur d'invasion.

2. Bactérie *E. coli* non pathogène selon la revendication 1, dans laquelle le facteur d'invasion est au moins un gène *inv* et au moins un gène *hlyA.*

3. Bactérie *E. coli* non pathogène selon l'une quelconque des revendications 1 ou 2, dans laquelle l'ARNsi interfère avec un ARNm NP, PA, PB1 ou PB2.

4. Bactérie *E. coli* non pathogène selon l'une quelconque des revendications 1 ou 2, dans laquelle l'ARNsi comprend une séquence choisie dans le groupe consistant en SEQ ID NO. 1, SEQ ID NO. 2, SEQ ID NO. 3, SEQ ID NO. 4, SEQ ID NO. 11, SEQ ID NO. 12, SEQ ID NO. 13 et SEQ ID NO. 14.

5. Bactérie *E. coli* non pathogène selon l'une quelconque des revendications 1 ou 2, dans laquelle le vecteur procaryote est un plasmide qui code pour un transcrit comprenant une séquence choisie dans le groupe consistant en SEQ ID NO. 5, SEQ ID NO. 7, SEQ ID NO. 15 et SEQ ID NO. 17.

6. Bactérie *E. coli* non pathogène selon l'une quelconque des revendications 1 ou 2, dans laquelle le promoteur est choisi dans le groupe consistant en un promoteur T7, un promoteur T3 et un promoteur SP6.

7. Bactérie *E. coli* non pathogène selon l'une quelconque des revendications 1 ou 2, dans laquelle l'ARNsi cible une séquence conservée, dans laquelle la séquence conservée est partagée par une pluralité de sous-types de grippe aviaire.

8. Bactérie *E. coli* non pathogène selon l'une quelconque des revendications 1 ou 2, dans laquelle le vecteur procaryote est un plasmide codant pour le gène *inv* et le gène *hlyA.*

9. Composition pharmaceutique comprenant la bactérie *E. coli* non pathogène selon l'une quelconque des revendications 1 à 8.

10. Composition pharmaceutique selon la revendication 9, dans laquelle la composition est appropriée pour une administration intranasale ou une pulvérisation sous forme d'aérosol approprié pour l'inhalation.

11. Composition pharmaceutique selon l'une quelconque des revendications 9 ou 10, comprenant une pluralité de populations de bactéries *E. coli* dans laquelle chaque population de *E. coli* est génétiquement modifiée pour exprimer une molécule d'ARNsi différente qui interfère avec une ou plusieurs molécules d'ARN viral de la grippe aviaire.

12. Composition pharmaceutique comprenant une première bactérie *E. coli* non pathogène selon l'une quelconque des revendications 1 à 8 et une seconde bactérie *E. coli* non-pathogène selon l'une quelconque des revendications 1 à 8, la première bactérie *E. coli* non pathogène comprenant un vecteur procaryote, ledit vecteur comprenant une molécule d'ADN codant pour un promoteur et un premier ARNsi, dans lequel le premier ARNsi codé interfère avec une ou plusieurs molécules d'ARN viral de la grippe aviaire et le promoteur contrôle la transcription du premier ARNsi, la seconde bactérie *E. coli* non pathogène comprenant un vecteur procaryote, ledit vecteur comprenant une molécule d'ARN codant pour un promoteur et un second ARNsi, dans laquelle le second ARNsi codé interfère avec une molécule d'ARN viral de la grippe aviaire différente et le promoteur contrôle la transcription du second ARNsi, les première et seconde bactéries dans un support pharmaceutiquement acceptable.

13. Composition pharmaceutique selon la revendication 12, dans laquelle la première séquence d'ARNsi codée comprend SEQ ID NO. 3 et la seconde séquence d'ARNsi codée comprend SEQ ID NO. 4.

14. Composition pharmaceutique selon l'une quelconque des revendications 9 à 13, pour une utilisation dans le traitement ou la réduction du risque de propagation de la grippe aviaire chez les oiseaux ou la volaille.

15. Vecteur procaryote selon la revendication 1, dans lequel le vecteur procaryote est un plasmide codant pour un ou plusieurs ARNsi, un promoteur pour contrôler la transcription des ARNsi et au moins un facteur d'invasion, dans lequel les ARNsi interfèrent avec l'ARNm d'un virus de la grippe aviaire.

16. Vecteur procaryote selon la revendication 15, dans lequel l'ARNsi interfère avec un ARNm NP, PA, PB1 ou PB2.

17. Vecteur procaryote selon la revendication 15, dans lequel l'ARNsi comprend une séquence choisie dans le groupe consistant en SEQ ID NO. 1, SEQ ID NO. 2, SEQ ID NO. 3, SEQ ID NO. 4, SEQ ID NO. 11, SEQ ID NO. 12, SEQ ID NO. 13 et SEQ ID NO. 14.


### SEQUENCE LISTING

<110> Colorado State University Research Foundation
<120> E. COLI MEDIATED siRNA SILENCING OF AVIAN INFLUENZA IN CHIKENS
<130> 2076.35.PR2C
<140> 14/604,252
   <141> 2015-01-23
<160> 18
<170> PatentIn version 3.5
<210> 1
   <211> 19
   <212> DNA
   <213> Influenza virus
<400> 1
   ggatcttatt tcttcggag 19
<210> 2
   <211> 19
   <212> DNA
   <213> Influenza virus
<400> 2
   gcaattgagg agtgcctga 19
<210> 3
   <211> 19
   <212> DNA
   <213> Influenza virus
<400> 3
   ctccgaagaa ataagatcc 19
<210> 4
   <211> 19
   <212> DNA
   <213> Influenza virus
<400> 4
   tcaggcactc ctcaattgc 19
<210> 5
   <211> 47
   <212> DNA
   <213> Artificial sequence
<220>
   <223> Influenza virus NP with shRNA sequence.
<400> 5
   ggatcttatt tcttcggagt tcaagagact ccgaagaaat aagatcc 47
<210> 6
   <211> 57
   <212> DNA
   <213> Artificial sequence
<220>
   <223> Influenza virus NP with shRNA and poly-T tail sequences.
<400> 6
   ggatcttatt tcttcggagt tcaagagact ccgaagaaat aagatccttt ttttttt 57
<210> 7
   <211> 47
   <212> DNA
   <213> Artificial sequence
<220>
   <223> Influenza PA with shRNA sequence.
<400> 7
   gcaattgagg agtgcctgat tcaagagatc aggcactcct caattgc 47
<210> 8
   <211> 57
   <212> DNA
   <213> Artificial sequence
<220>
   <223> Influenza virus PA with shRNA and poly-T tail sequences.
<400> 8
   gcaattgagg agtgcctgat tcaagagatc aggcactcct caattgcttt ttttttt 57
<210> 9
   <211> 10
   <212> DNA
   <213> Artificial sequence
<220>
   <223> Hairpin loop sequence.
<400> 9
   ttcaagagat 10
<210> 10
   <211> 10
   <212> DNA
   <213> Artificial sequence
<220>
   <223> Poly-T Tail sequence.
<400> 10
   tttttttttt 10
<210> 11
   <211> 19
   <212> DNA
   <213> Influenza virus
<400> 11
   gatctgttcc accattgaa 19
<210> 12
   <211> 19
   <212> DNA
   <213> Influenza virus
<400> 12
   cgggactcta gcatactta 19
<210> 13
   <211> 19
   <212> DNA
   <213> Influenza virus
<400> 13
   ttcaatggtg gaacagatc 19
<210> 14
   <211> 19
   <212> DNA
   <213> Influenza virus
<400> 14
   tcaggcactc ctcaattgc 19
<210> 15
   <211> 47
   <212> DNA
   <213> Artificial sequence
<220>
   <223> Influenza virus PB1 with shRNA sequence.
<400> 15
   gatctgttcc accattgaat tcaagagatt caatggtgga acagatc 47
<210> 16
   <211> 57
   <212> DNA
   <213> Artificial sequence
<220>
   <223> Influenza virus PB1 with shRNA and poly-T tail sequences.
<400> 16
   gatctgttcc accattgaat tcaagagatt caatggtgga acagatcttt ttttttt 57
<210> 17
   <211> 47
   <212> DNA
   <213> Artificial sequence
<220>
   <223> Influenza virus PB2 with shRNA sequence.
<400> 17
   cgggactcta gcatacttat tcaagagata agtatgctag agtcccg 47
<210> 18
   <211> 57
   <212> DNA
   <213> Artificial sequence
<220>
   <223> Influenza PB2 sequence with shRNA and poly-T tail sequences.
<400> 18
   cgggactcta gcatacttat tcaagagata agtatgctag agtcccgttt ttttttt 57